# EUROPÄISCHE PATENTSCHRIFT

(11) **EP 2 632 607 B1**
(45) Veröffentlichungstag und Bekanntmachung des Hinweises auf die Patenterteilung: **21.02.2018**
(21) Anmeldenummer: 11778554.3
(22) Anmeldetag: 26.10.2011
(51) Int. Cl.: B05C 17/005

(54) **DOPPELKARTUSCHE, MISCHER HIERFÜR UND KOMBINATION AUS DOPPELKARTUSCHE UND MISCHER**
DOUBLE CARTRIDGE, MIXER THEREFOR AND COMBINATION OF DOUBLE CARTRIDGE AND MIXER
CARTOUCHE DOUBLE, MÉLANGEUR DESTINÉ À CELLE-CI ET COMBINAISON CARTOUCHE DOUBLE ET MÉLANGEUR

(30) Priorität: 24.08.2011 DE 102011111046; 04.02.2011 DE 202011002407 U; 26.10.2010 DE 102010049378
(43) Veröffentlichungstag der Anmeldung: 04.09.2013
(73) Patentinhaber: Kettenbach GmbH & CO. KG, 35713 Eschenburg (DE)
(72) Erfinder: BUBLEWITZ, Alexander, 35745 Herborn (DE); REBER, Jens-Peter, 58540 Meinerzhagen (DE)
(74) Vertreter: Keil & Schaafhausen Patent- und Rechtsanwälte PartGmbB
(86) Internationale Anmeldenummer: PCT/EP2011/068784
(87) Internationale Veröffentlichungsnummer: WO 2012/055926

(56) Entgegenhaltungen:
- DE-U1-202006 004 738
- US-A- 4 981 241
- US-A1- 2007 095 865
- US-A1- 2010 089 949
- US-A1- 2010 102 088

## Beschreibung

Die Erfindung betrifft eine Kombination aus einer Doppelkartusche mit einem Mischer, wobei die Doppelkartusche zwei Vorratsbehälter aufweist, die jeweils ein vorderes Ende mit einem Auslassstutzen aufweisen. Die Längsachsen der Auslassstutzen sollen zumindest im Wesentlichen parallel zueinander verlaufen. Auf ihren Außenmantelflächen ist jeweils wenigstens ein Vorsprung angeordnet. Der Mischer weist ein Gehäuse auf, das einen Mischraum definiert, wobei an einem Auslassende des Gehäuses eine Auslassöffnung vorgesehen ist. An seinem gegenüberliegenden Einlassende sind zwei in den Mischraum mündende Einlassstutzen vorgesehen, deren Längsachsen zumindest im Wesentlichen parallel zueinander verlaufen. Weiter betrifft die Erfindung einen Mischer mit einem Gehäuse, das einen Mischraum definiert, wobei an einem Auslassende des Gehäuses eine Auslassöffnung und an seinem gegenüberliegenden Einlassende zwei in den Mischraum mündende Einlassstutzen vorgesehen sind, deren Längsachsen zumindest im Wesentlichen parallel zueinander verlaufen. Insbesondere ist der Mischer mit der Doppelkartusche verbindbar, um die in der Doppelkartusche aufgenommenen Substanzen miteinander zu vermischen und das Gemisch auszubringen. Hierzu können die Einlassstutzen des Mischers mit den Auslassstutzen der Doppelkartusche verbunden werden.

Derartige Systeme werden bspw. im Dentalbereich zur Aufnahme und Verarbeitung von Komponenten für Abfüllmaterialien, Füllmaterialien, Klebstoffe oder dergleichen verwendet. Die Komponenten werden dabei in den Vorratsbehältern getrennt voneinander gelagert und können in einem entsprechenden Austraggerät mittels Ausbringkolben oder Stempeln durch die Auslassstutzen in den Mischer ausgebracht werden. Bei dem Mischer kann es sich um einen statischen Mischer handeln oder einen dynamischen Mischer, bei welchem ein Mischerelement innerhalb des Gehäuses mittels einer Welle des Ausbringgerätes angetrieben bewegt wird.

Bei den meisten am Markt verfügbaren Ausbringgeräten werden die Kartuschen derart geneigt gehalten, dass die Auslassstutzen schräg nach unten weisen. Dies erleichtert das Befüllen bspw. eines Abformlöffels mit den aus dem Mischer austretenden gemischten Komponenten. Gleichzeitig bringt diese Anordnung jedoch mit sich, dass die Auslassstutzen und ggf. die Mischerwelle für Benutzer schlecht einsehbar sind, so dass das vor jeder Benutzung erforderliche Aufsetzen eines neuen Mischers auf die Doppelkartusche teilweise schwierig ist.

So werden in der EP 2 335 641 A1 eine Doppelkartusche und ein mit dieser verbindbarer statischer Mischer beschrieben, bei denen die Befestigung zwischen dem Mischer und der Doppelkartusche über eine Bajonett-Verbindung erfolgt, die auf einer Kappe vorgesehen ist, welche ein Ankopplungselement, das im Wesentlichen aus den Einlassstutzen und einer Mischwendel besteht, und das Mischergehäuse umgreift und relativ zu diesen verdrehbar ist. Bei diesem Mischer ist das Ankopplungselement drehbar in dem Mischergehäuse gelagert. Eine ähnliche Lösung ist aus der WO 2011/041917 A1 bekannt, wobei die Bajonettverbindungsmittel eine schräg gestellte Drehführung aufweisen, die bewirkt, dass während des Herstellens der Verbindung ein zwangsläufiges Heranführen des Mischers an die Kartusche erfolgt und dass während des Lösens der Verbindung ein zwangsläufiges Abheben des Mischers von der Kartusche erfolgt. Vor diesem Eingreifen dieser (Bajonett-)Verbindungsmittel müssen diese zunächst ineinander gesteckt werden, wobei Kodierungsmittel sicherstellen, dass das Mischergehäuse definiert zu der Kartusche ausgerichtet ist. Mit anderen Worten muss der Mischer zunächst so weit auf die Kartusche aufgesetzt werden, dass die Einlassöffnungen des Mischers mit den Auslassstutzen der Kartusche verbunden sind, bevor der Mischer durch eine Drehung der bspw. in der Art einer Überwurfmutter ausgestalteten Bajonettbefestigungsmittel mit der Kartusche verriegelt werden kann.

Eine definierte Ausrichtung eines Mischers relativ zu einer Kartusche ist auch Gegenstand der EP 0 730 913 A1, die ähnliche Kodierungsmittel wie in der EP 2 335 641 A1 oder der WO 2011/041917 A1 aufweist. Diese Kodierungsmittel sind allerdings allenfalls dazu geeignet, ein Mischergehäuse bzw. einen Bajonettring relativ zu der Kartusche auszurichten, ohne dass hierdurch zwangsläufig auch die Auslässe der Kartusche mit den Einlässen des Mischers in Überdeckung gebracht werden können.

So erfordert die Verbindung zwischen dem Mischer und der Kartusche bspw. bei den Lösungen nach der EP 2 335 641 A1 oder der WO 2011/041917 A1 eine visuelle Kontrolle und ein exaktes Aufsetzen des Mischers in einer vorbestimmten Position. Dies wird teilweise als mühsam und unpraktisch empfunden. Zudem ist bei den Mischern nach dem Stand der Technik schon während der Montage des Mischers eine spezielle Ausrichtung des Ankopplungselements relativ zu dem Mischergehäuse erforderlich, da der Mischer sonst nicht auf die Kartusche aufgesetzt werden kann. Dies erfordert einen zusätzlichen kostenproduzierenden Montageschritt einschließlich der erforderlichen Qualitätskontrolle. Wenn das Ankopplungselement trotz dieser Vorausrichtung versehentlich relativ zu dem Mischergehäuse verdreht wird, kann der Mischer nicht mehr auf der Kartusche befestigt werden, ohne zuvor manuell diese beiden Bauteile wieder zueinander auszurichten. Zudem erweisen sich die aus der EP 2 335 641 A1 oder der WO 2011/041917 A1 bekannten Bajonettbefestigungsmittel, die einander nur an zwei Stellen hintergreifen, insbesondere bei hohem Ausbringdruck als nachteilig, der beim Austragen pastöser Massen und/oder bei hoher Ausbringgeschwindigkeit entsteht.

Weiter existiert eine Kombination aus einem Mischer, einem Bajonettring in der Art einer Überwurfmutter und einer Kartusche, die von der Sulzer Mixpac AG (CH-9469 Haag) unter den Produktnummern MBD 381-05-00 (Mischer), BBD 381-00-11 (Bajonettring) und CBD 381-05-58 (Kartusche) angeboten wird. Eine weitgehend ähnliche Kombination ist auch in der EP 1 943 012 B1 beschrieben. Hierbei weist der Mischer einen Deckel bzw. ein Ankopplungselement, das im Wesentlichen aus den Einlassstutzen und einer Mischwendel besteht, ein Mischergehäuse und ein drehbar in dem Ankopplungselement gelagertes Mischelement auf. Von der der Kartusche zugewandten Seite des Ankopplungselements ragt parallel zu den Einlassstutzen des Mischers ein Kodierungselement weg, welches mit Spiel in eine Ausnehmung einer Platte eingreifen kann, die an den Auslassstutzen der Kartusche befestigt ist. Die Länge der Ein- und Auslassstutzen sowie des Kodierungselements sind dabei so bemessen, dass beim Aufsetzen des Mischers auf die Kartusche die Einlassstutzen bereits in die Auslassstutzen eingesteckt sind, bevor das Kodierungselement die Ausnehmung in der Platte erreicht. Weder das Kodierungselement noch die Ausnehmung in der Platte können daher eine Führung des Mischers beim Aufsetzen auf die Kartusche bewirken, sondern verhindern lediglich, dass der Mischer mittels des Bajonettrings an der Kartusche verriegelt werden kann, wenn der Mischer um 180° verdreht auf die Kartusche aufgesetzt ist, d.h. die falschen Ein- und Auslassstutzen ineinandergreifen. Unabhängig davon erlaubt auch das große Spiel zwischen dem Kodierungselement und der Ausnehmung in der Platte keine Führung des Mischers an der Kartusche.

Die EP 1 440 737 A1 zeigt ein System, das Kodierungsmittel aufweist, die als Leisten gestaltet sind, welche in Ausnehmungen eingreifen, die beabstandet von den Kartuschenauslässen angeordnet sind. Die Kodierungsmittel können nur ein falsches Aufsetzen des Mischers verhindern, ohne jedoch das Aufsetzen des Mischers ohne optische Kontrolle zu erleichtern.

Die US 2007/0095865 A1 behandelt einen Dichtstoffbehälter mit einer Spitze. Letztere hat ein Außengewinde und kann damit am Dichtstoffbehälter befestigt werden. Das System ist nur zur Aufnahme und zur Ausbringung einer Komponente geeignet, ein Mischer ist nicht enthalten. Das Außengewinde dient allein der Befestigung, darüber hinaus sind keine Maßnahmen zur Führung beziehungsweise Positionierung der Spitze relativ zum Dichtstoffbehälter bei der Montage genannt.

In DE 20 2006 004 738 U1 wird eine Vorrichtung für das Mischen zweier Fluide beschrieben. Dabei ist ein Mischer auswechselbar an einem Adapterelement angeordnet, welches wiederum auswechselbar an einem Spritzenkörper angeordnet ist. In dem Adapterelement findet jedoch keine Vermischung der Fluide statt. Die am Adapterelement positionierten Rastmittel dienen lediglich zur Befestigung des Adapterelementes an dem Spritzenkörper. Sie stehen nicht direkt mit dem Mischer in Kontakt und bieten keine mechanische Führung zur Vereinfachung der Positionierung des Mischers am Spritzenkörper.

Der vorliegenden Erfindung liegt demgegenüber die Aufgabe zugrunde, eine Doppelkartusche sowie einen Mischer bereitzustellen, die leichter miteinander verbindbar sind.

Diese Aufgabe wird erfindungsgemäß im Wesentlichen mit einer Kombination aus einer Doppelkartusche mit einem Mischer mit den Merkmalen des Anspruchs 1 sowie einem Mischer mit den Merkmalen des Anspruchs 7 gelöst. Unter einer solchen Kombination bzw. einem Set wird eine Ausbringanordnung verstanden, die aus einer Doppelkartusche und einem lösbar mit dieser verbindbaren oder verbundenen Mischer besteht. Der Erfindung liegt dabei der Gedanke zugrunde, unabhängig von den miteinander zu verbindenden Auslassstutzen und Einlassstutzen der Doppelkartusche bzw. des Mischers eine Führung vorzusehen, die die exakte

Positionierung des Mischers relativ zu der Doppelkartusche erleichtert. Mit anderen Worten soll die Führung sicherstellen, dass die Einlassstutzen des Mischers in die Auslassstutzen der Doppelkartusche eingesteckt werden können, auch wenn die Sicht auf die Auslassstutzen bspw. in Folge der Anordnung des Austraggerätes erschwert oder nicht vorhanden ist. Somit ist auch ein intuitives rein taktiles Finden der richtigen Mischerposition möglich. Dabei wird es besonders bevorzugt, wenn gleichzeitig auch eine ggf. vorgesehene Mischerwelle des Austraggerätes mit dem Mischer verbunden werden kann.

Hierzu ist es erfindungsgemäß vorgesehen, dass der wenigstens eine Vorsprung derart angeordnet ist, dass zwischen den Vorsprüngen ein Führungskanal ausgebildet wird, der sich zumindest im Wesentlichen parallel zu den Längsachsen der Auslassstutzen erstreckt. Abhängig von der Größe und Ausrichtung der Vorsprünge kann der Führungskanal auch in seiner Erstreckung parallel zu den Längsachsen der Auslassstutzen sehr kurz sein, bspw. wenn die Vorsprünge etwa punktförmig sind. Dies schließt auch Ausführungsformen ein, bei denen die Auslassstutzen der Kartusche in eine Platte münden oder von einer Platte umgeben sind, wobei eine Öffnung in einer solchen Platte dann den Führungskanal definiert. In diesem Fall definiert die Dicke der Platte die Länge des Führungskanals. Auf der dem Mischer abgewandten Seite der Platte können zusätzliche Führungselemente vorgesehen sein, um die Länge des Führungskanals zu vergrößern. Weiter ist die Erfindung nicht auf die Ausgestaltung eines einzigen Führungskanals beschränkt, vielmehr können mehrere Führungskanäle ausgebildet sein, in die jeweils eine Führungsrippe oder dgl. des Mischers eingreifen kann. Diese letztgenannte Ausgestaltung ist besonders dann geeignet, wenn die Auslassstutzen der Kartusche in eine Platte münden oder von einer Platte umgeben sind.

Eine Doppelkartusche ist im Sinne dieser Erfindung bspw. eine Einheit aus zwei einstückig miteinander verbundenen Behältern. Alternativ hierzu kann eine Doppelkartusche auch durch zwei einzelne Behälter, insbesondere jeweils mit steifen Kappen versehene flexible Schlauchbeutel, gebildet sein, wobei diese einzelnen Behälter miteinander verbindbar sind. Dies kann bspw. durch Einführen der Behälter in zwei miteinander verbundene Rohre erfolgen, die als Stützkartusche dienen. Hierbei wird es bevorzugt, wenn die einzelnen Behälter bzw. deren Kappen, an denen jeweils ein Auslassstutzen für die in dem jeweiligen Schlauchbeutel oder dgl. enthaltenen Substanzen vorgesehen ist, so zueinander ausgerichtet werden können, insbesondere durch entsprechende Anordnung in der Stützkartusche, dass die jeweils auf der Außenmantelfläche der Auslassstutzen vorgesehenen Vorsprünge derart angeordnet sind, dass zwischen den Vorsprüngen ein sich parallel zu den Längsachsen der Auslassstutzen erstreckender Führungskanal ausgebildet wird.

Bei einer Ausführungsform einer Doppelkartusche kann wenigstens einer der Vorsprünge als ein sich zumindest im Wesentlichen parallel zu den Längsachsen der Auslassstutzen erstreckender Führungssteg ausgebildet sein. Unter einem Führungssteg wird hierbei ein Vorsprung verstanden, dessen Haupterstreckungsrichtung im Wesentlichen parallel zu der Längsachse des jeweiligen Auslassstutzens ausgerichtet ist. Derartige Führungsstege erlauben eine besonders gute Führung eines mit der Doppelkartusche verbindbaren Mischers, ohne dass die Gefahr eines Verkantens des Mischers besteht.

Alternativ oder zusätzlich hierzu kann wenigstens einer der Vorsprünge als eine sich im Wesentlichen rechtwinklig zu den Längsachsen der Auslassstutzen erstreckende Rastnocke ausgebildet sein. Dies ermöglicht es, dass der Vorsprung zusätzlich zu seiner Funktion der Führung des Mischers auch eine Befestigung des Mischers und/oder eines Verschlusselements für die Doppelkartusche, bspw. mittels eines Schnapp- oder Rastverschlusses, dient.

Die Vorsprünge sind vorzugsweise auf der während der Benutzung der Doppelkartusche dem Benutzer zugewandten Seite der Auslassstutzen angeordnet. So kann auf einer ersten Seite der Doppelkartusche ein Sicherungsbügel zum Fixieren des Mischers an den Auslassstutzen vorgesehen sein, wobei die Vorsprünge dann ebenfalls auf dieser ersten Seite der Auslassstutzen angeordnet sind.

Hinsichtlich der Befüllbarkeit, der Stabilität während des Ausbringens der Komponenten sowie hinsichtlich der Handhabbarkeit hat es sich als besonders zweckmäßig erwiesen, wenn die Doppelkartusche zusätzlich zu den Vorratsbehältern eine Stützkartusche aufweist, in welcher die Vorratsbehälter vorzugsweise lösbar aufgenommen sind. Die Stützkartusche kann dabei aus zwei, insbesondere starr miteinander verbundenen, Rohren aus Metall oder einem Faserverbundwerkstoff gebildet sein. Hierdurch ist es möglich, die Vorratsbehälter vergleichsweise dünnwandig und aus einem kostengünstigen und/oder hinsichtlich der Verarbeitbarkeit vorteilhaften Material herzustellen, da zumindest die in radialer Richtung wirkenden Kräfte im Wesentlichen von der Stützkartusche aufgenommen werden können.

In Weiterbildung dieses Gedankens ist es vorgesehen, dass die Vorratsbehälter voneinander beabstandet und nur an ihrem hinteren Ende einstückig miteinander durch eine Brücke verbunden sind. Auf diese Weise ist es möglich, die Vorratsbehälter der Doppelkartusche von einem in Ausbringrichtung hinteren Ende in die Stützkartusche einzuführen, bis die Auslassstutzen auf dem gegenüberliegenden Ende aus der Stützkartusche vorstehen. Die Brücke, mit welcher die beiden Vorratsbehälter verbunden sind, dient dabei gleichzeitig zum Abstützen der beim Ausbringen der Komponenten aus der Doppelkartusche auf die Vorratsbehälter wirkenden axialen Belastungen. Zusätzlich können die Vorratsbehälter auch auf dem in Ausbringrichtung vorderen Ende in der Stützkartusche abgestützt sein.

Erfindungsgemäß ist die Kartusche mit einer Platte oder Scheibe versehen, die an den Auslassstutzen angeordnet ist. Vorzugsweise münden die Auslassstutzen in diese Platte bzw. schließen bündig mit dieser ab. Alternativ können die Auslassstutzen in Richtung des Mischers auch über die Platte herausragen. Die Platte oder Scheibe bildet somit bspw. einstückig miteinander verbundene Vorsprünge, zwischen denen ein Führungskanal als eine Öffnung in der Platte ausgebildet wird. Zusätzlich können auf dieser Platte oder Scheibe weitere Vorsprünge oder Stege vorgesehen sein, die ebenfalls zur Führung des Mischers beitragen. Diese zusätzlichen Stege können unmittelbar mit den Auslassstutzen verbunden sein oder geringfügig von diesen beabstandet auf der Platte angeordnet sein. Letzteres kann beim Spritzguss Verziehungen vermeiden.

In Weiterbildung dieser Ausführungsform ist vorzugsweise ein Ring oder dgl. flanschartiger Rand an der Platte vorgesehen. Dieser Ring ragt vorzugsweise in Richtung des Mischers von der Platte weg und kann dabei eine Aufnahme für das kartuschenseitige Ende des Mischers bilden.

Eine besonders einfache und gleichzeitig stabile Befestigung des Mischers an der Kartusche kann dadurch erreicht werden, dass der Ring auf seiner Innenseite erfindungsgemäß ein Gewinde aufweist, in welches ein Außengewinde des Mischers einschraubbar ist.

Unabhängig von den zuvor genannten Merkmalen liegt ein besonderer Aspekt der vorliegenden Erfindung darin, dass an der Kartusche Mittel vorgesehen sind, die bei einer relativen Bewegung, insbesondere einer relativen Drehbewegung, des Mischers oder Mischergehäuses zu der Kartusche den Mischer von der Kartusche abheben bzw. ablösen. Dies ist bspw. dann vorteilhaft, wenn der Mischer nach Aushärten des Gemisches an der Kartusche anhaftet bzw. festklebt. Derartige Mittel zum Abheben des Mischers können bspw. eine Gewindeverbindung zwischen Mischergehäuse und Kartusche umfassen, so dass der Mischer bei einer Verdrehung des Mischergehäuses relativ zu der Kartusche auch eine axiale Relativbewegung ausführt. Alternativ oder zusätzlich hierzu können auch eine oder mehrere Rampen an dem Mischer und/oder der Kartusche vorgesehen sein, die das Abheben des Mischers von der Kartusche erleichtern. Insbesondere können solche Rampen an einer Platte vorgesehen sein, in die die Auslassstutzen münden, und/oder an einem diese Platte umgebenden Ring.

Der erfindungsgemäße Mischer weist an seinem Einlassende wenigstens eine Führungsrippe auf, die sich zumindest im Wesentlichen parallel zu den Längsachsen der Einlassstutzen erstreckt. Mit anderen Worten ragt von dem hinteren Ende des Mischers, welches in Benutzung der Doppelkartusche zugewandt ist, wenigstens eine Führungsrippe weg, welche zwischen den Vorsprüngen auf den Auslassstutzen der Doppelkartusche derart geführt werden kann, dass die Einlassstutzen des Mischers die Auslassstutzen der Doppelkartusche treffen.

Wenn der erfindungsgemäße Mischer ein dynamischer Mischer ist, dessen bewegbares Mischelement mit einer Mischerwelle des Ausbringgerätes verbunden wird, hat es sich als besonders vorteilhaft erwiesen, wenn in der Führungsrippe eine, insbesondere etwa U-förmige, Aussparung vorgesehen ist. Diese erstreckt sich vorzugsweise bis zu einer dem Auslassende abgewandten Kante der Führungsrippe. Die Aussparung verhindert dabei, dass die Führungsrippe die Mischerwelle verdeckt, sodass es einem Benutzer möglich ist, während des Aufsetzens des Mischers weiterhin die richtige Ausrichtung der Mischerwelle zu dem Mischer zu überprüfen. Alternativ zu der Ausgestaltung einer Führungsrippe mit einer Aussparung ist es auch möglich, dass sich zwei voneinander beabstandete Führungsrippen von dem rückseitigen Ende des Mischers wegerstrecken, so dass zwischen diesen beiden Führungsrippen eine Aussparung verbleibt, durch welche die Mischerwelle sichtbar ist.

Wenn die wenigstens eine Führungsrippe die Einlassstutzen des Mischers überragt, ist eine Führung des Mischers relativ zu der Doppelkartusche möglich, noch bevor die Einlassstutzen des Mischers mit den Auslassstutzen der Doppelkartusche in Kontakt treten. Auf diese Weise ist es möglich, dass ein Benutzer den Mischer lediglich derart ausrichten muss, dass die Führungsrippe in den zwischen den Vorsprüngen gebildeten Führungskanal eingelegt wird, um den Mischer auf die Doppelkartusche aufstecken zu können. Dies erleichtert die Handhabung des Mischers erheblich.

Die Führungsrippe des Mischers kann auch gewölbt oder gebogen ausgestaltet sein. Bspw. kann sich die Führungsrippe in einem Querschnitt senkrecht zu der Mischerachse etwa kreissegmentförmig um die Mischerachse erstrecken.

Unabhängig von den zuvor genannten Merkmalen wird es bevorzugt, wenn der Mischer ein Gehäuse aufweist, welches relativ zu einem mit den Einlassstutzen versehenen Deckelteil des Mischers drehbar ist. Bei dieser Ausgestaltung kann das Mischergehäuse erfindungsgemäß mit einem Außengewinde versehen sein, um ein Einschrauben des Mischers, bspw. in einen Ring an der Kartusche, zu ermöglichen, wobei gleichzeitig die Einlassstutzen und die Auslassstutzen ineinandergreifen.

Ein weiterer Aspekt der vorliegenden Erfindung liegt darin, dass an dem Mischer Mittel vorgesehen sind, die bei einer relativen Bewegung, insbesondere einer relativen Drehbewegung, des Mischers oder Mischergehäuses zu der Kartusche den Mischer von der Kartusche abheben bzw. ablösen. Derartige Mittel zum Abheben des Mischers können bspw. eine Gewindeverbindung zwischen Mischergehäuse und Kartusche umfassen und/oder Rampen, die mit entsprechenden Gegenkonturen der Kartusche zusammenwirken können.

Dabei ist die Breite des zwischen den Vorsprüngen gebildeten Führungskanals vorzugsweise so auf die Breite der Führungsrippe bzw. den Abstand der Führungsrippen im Falle zweier Führungsrippen abgestimmt, dass der Mischer durch die Führungsrippe(n) und den Führungskanal relativ zu der Doppelkartusche geführt auf diese aufsetzbar ist. Mit anderen Worten treffen sich bei einer erfindungsgemäßen Ausbringanordnung zunächst die Führungsrippe(n) des Mischers und der wenigstens eine Führungskanal der Kartusche, bevor beim Aufsetzen eines Mischers auf die Kartusche auch die Ein- und Auslassstutzen ineinandergreifen.

Die Erfindung wird nachfolgend anhand eines Ausführungsbeispiels und unter Bezugnahme auf die Zeichnung näher erläutert. Dabei bilden alle beschriebenen und/oder bildlich dargestellten Merkmale für sich den Gegenstand der Erfindung, unabhängig von ihrer Zusammenfassung in den Ansprüchen oder deren Rückbezügen. Es zeigen schematisch:
- Fig. 1: in Explosionsansicht die Komponenten einer Kartusche sowie einem Mischer,
- Fig. 2: in Perspektivansicht die Kartusche nach Fig. 1,
- Fig. 3: in teilweise geschnittener Darstellung die Kartusche nach Fig. 1,
- Fig. 4: in Perspektivansicht die Kartusche nach Fig. 1 mit aufgesetztem Verschlussstopfen,
- Fig. 5a: ein Detail der Kartusche nach Fig. 3 während des Aufsteckens eines Mischers,
- Fig. 5b: ein Detail der Kartusche nach Fig. 3 mit aufgestecktem Mischer,
- Fig. 6: in Schnittansicht die Ausbringkolben der Kartusche nach Fig. 1,
- Fig. 7: in Perspektivansicht einen Drehverschluss eines Ausbringkolbens nach Fig. 6
- Fig. 8: in einer weiteren Perspektivansicht den Drehverschluss nach Fig. 7,
- Fig. 9: in Perspektivansicht einen Verschlussstopfen nach einer weiteren Ausführungsform,
- Fig. 10: in Seitenansicht den Verschlussstopfen nach Fig. 9,
- Fig. 11: in Perspektivansicht den auf eine Doppelkartusche aufgesetzten den Verschlussstopfen nach Fig. 9,
- Fig. 12a - h: in vergrößerter Perspektivansicht ein Detail des Mischers,
- Fig. 13: eine zweite Ausführungsform einer Doppelkartusche,
- Fig. 14: eine dritte Ausführungsform einer erfindungsgemäßen Doppelkartusche,
- Fig. 15: in Perspektivansicht eine vierte Ausführungsform eines Mischers mit einer Kartusche vor dem Aufsetzen des Mischers,
- Fig. 16: in Perspektivansicht die Ausführungsform nach Fig. 15 nach dem Aufsetzen des Mischers,
- Fig. 17: in Perspektivansicht die Kartusche nach Fig. 15 vor dem Aufsetzen des Mischers,
- Fig. 18: in Schnittansicht den Mischer nach Fig. 15 während des Aufsetzens auf die Kartusche,
- Fig. 19: in Schnittansicht den Mischer nach Fig. 15 nach dem Aufsetzen auf die Kartusche,
- Fig. 20: in Perspektivansicht eine fünfte Ausführungsform eines Mischers mit einer Kartusche vor dem Aufsetzen des Mischers,
- Fig. 21: in Perspektivansicht eine sechste Ausführungsform eines Mischers mit einer Kartusche vor dem Aufsetzen des Mischers,
- Fig. 22: in Perspektivansicht eine siebte Ausführungsform eines Mischers mit einer Kartusche während des Einschraubens des Mischers,
- Fig. 22A: vergrößert das Detail A aus Fig. 22,
- Fig. 23: in Perspektivansicht den Mischer nach Fig. 22 während des Ausschraubens des Mischers,
- Fig. 23A: vergrößert das Detail A aus Fig. 23,
- Fig. 24: in Perspektivansicht eine achte Ausführungsform eines Mischers mit einer Kartusche vor dem Aufsetzen des Mischers,
- Fig. 25: in Perspektivansicht eine neunte Ausführungsform eines Mischers mit einer Kartusche vor dem Aufsetzen des Mischers,
- Fig. 26: in Perspektivansicht eine zehnte Ausführungsform eines Mischers mit einer Kartusche vor dem Aufsetzen des Mischers,
- Fig. 27: in teilweise geschnittener Ansicht einen auf eine Kartusche aufgesetzten Verschluss,
- Fig. 27A: vergrößert das Detail A aus Fig. 27,
- Fig. 28: in Perspektivansicht eine elfte Ausführungsform eines Mischers mit einer Kartusche vor dem Aufsetzen des Mischers,
- Fig. 29: in einer weiteren Perspektivansicht den Mischer und die Kartusche nach Fig. 28,
- Fig. 30: in Schnittansicht eine zwölfte Ausführungsform eines Mischers, und
- Fig. 31: in Perspektivansicht die Mischerwelle des Mischers nach Fig. 30.

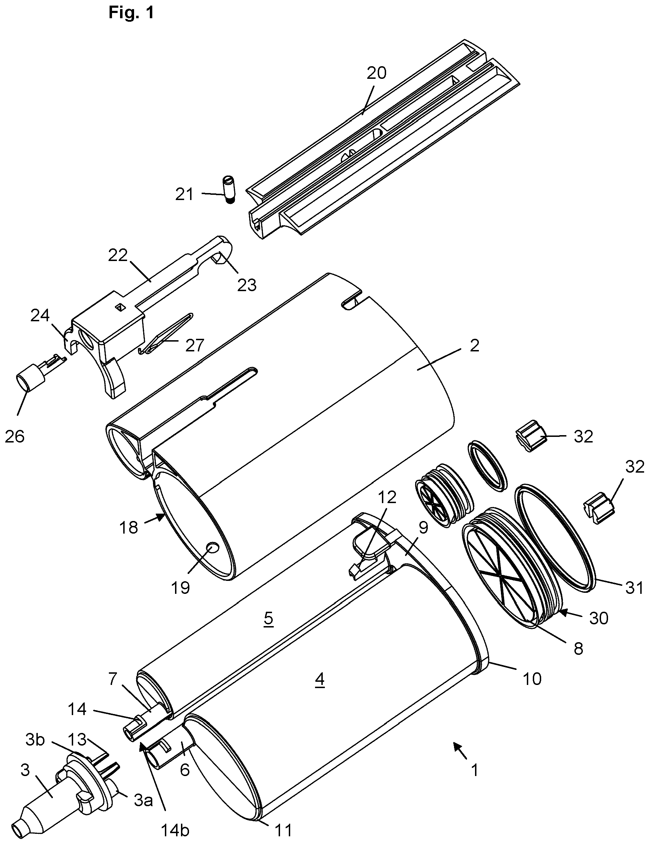

In den Figuren 1 bis 13 sind alternative Beispiele dargestellt, die nicht unter die Ansprüche fallen. Die in Figur 1 dargestellte Kartuschenanordnung weist im Wesentlichen eine Doppelkartusche 1 sowie eine Stützkartusche 2 auf. Die Doppelkartusche 1 ist dabei mit einem Mischer 3 lösbar verbindbar.

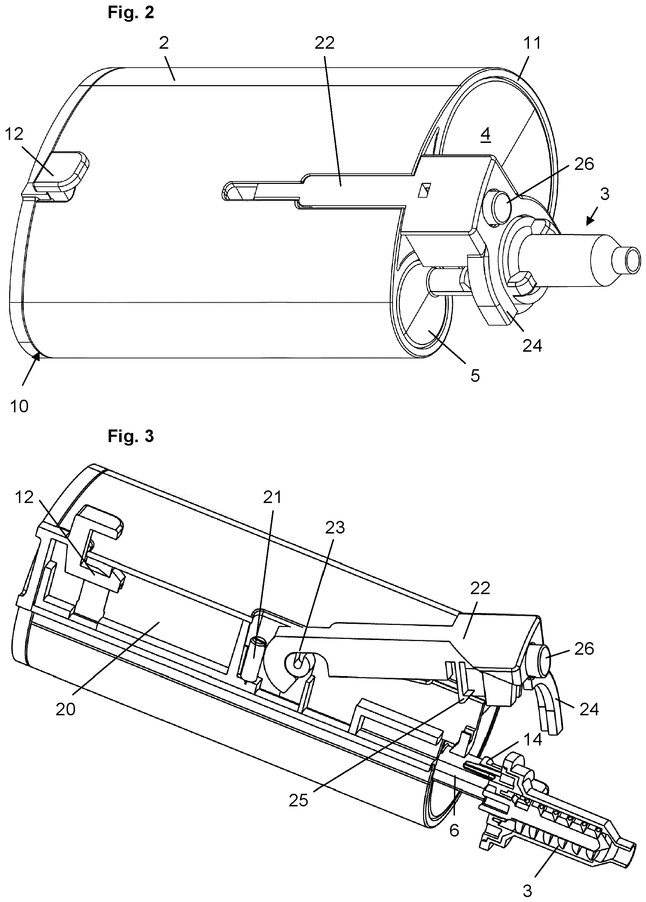

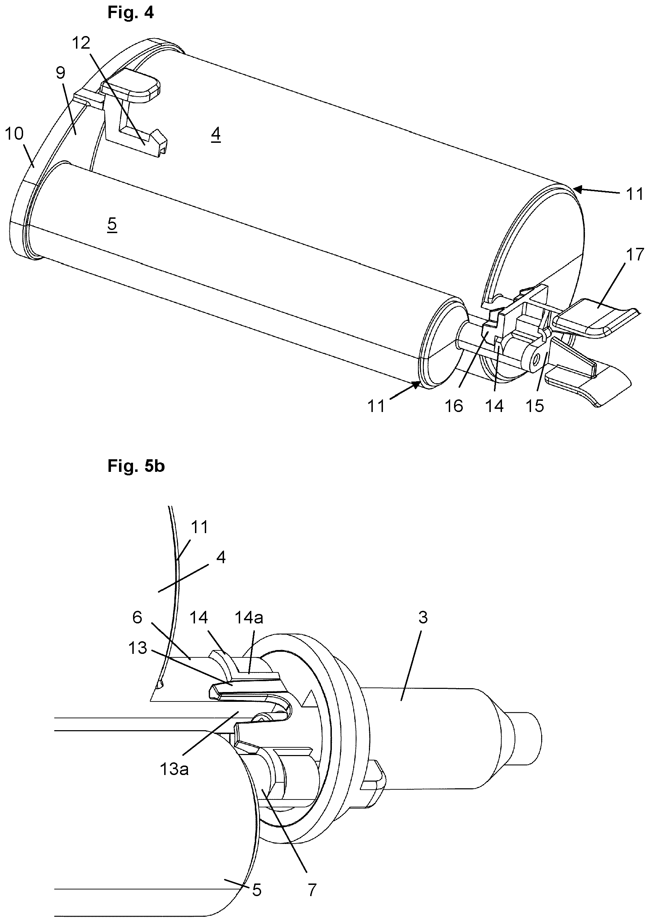

Wie auch aus den Figuren 3 und 4 ersichtlich ist, besteht die Doppelkartusche 1 im Wesentlichen aus zwei voneinander getrennten Vorratsbehälter 4, 5, deren Gehäuse im Wesentlichen zylindrisch ausgebildet sind. Das in Figur 1 links dargestellte, vordere Ende der Vorratsbehälter 4, 5 ist durch eine stirnseitige Wand geschlossen, die ausreichend dick dimensioniert ist, um auch hohen Kräften beim Austragen der Komponenten standzuhalten. Aus dieser stirnseitigen Wand steht jeweils ein Auslassstutzen 6 bzw. 7 hervor, der eine Auslassöffnung des jeweiligen Vorratsbehälters bildet. Die Auslassstutzen 6, 7 sind in der dargestellten Ausführungsform dicht nebeneinander liegend, d.h. nicht mittig in der stirnseitigen Wand der Vorratsbehälter, angeordnet. Dies ermöglicht es, den Mischer 3 vergleichsweise kompakt zu gestalten, da dessen Einlasstutzen 3a, 3b nicht weit voneinander beabstandet sein müssen. Auf der gegenüberliegenden, hinteren Seite sind die Vorratsbehälter 4, 5 offen, so dass unten näher erläuterte Ausbringkolben 8 zur Abdichtung in die Vorratsbehälter eingesetzt werden können. Die Ausbringkolben 8 dienen gleichzeitig dazu, die in den Vorratsbehältern 4, 5 aufgenommenen Stoffe, bspw. Komponenten eines Abformmaterials oder dgl. durch die Auslassstutzen 6, 7 auszubringen.

An ihrem hinteren Ende sind die Vorratsbehälter 4, 5 über eine Brücke 9 miteinander verbunden. Die Brücke 9 ist einstückig mit einem flanschartigen Rand 10 ausgebildet, welcher über das hintere Ende der Vorratsbehälter 4, 5 hinausragt. Am vorderen Ende der beiden Vorratsbehälter 4, 5 ist in der stirnseitigen Wand eine umlaufende Nut 11 vorgesehen sein, die als Anschlag in der Stützkartusche 2 dient.

Die Brücke 9 ist in der dargestellten Ausführungsform mit einem Rasthebel 12 versehen, welcher einstückig mit der Brücke 9 und den Vorratsbehältern 4, 5 ausgebildet ist. An dem in Ausbringrichtung vorderen Ende des Rasthebels 12 ist ein Rasthaken vorgesehen, dessen Funktion unten näher erläutert wird.

Die Auslassstutzen 6, 7 der Vorratsbehälter 4, 5 sind mit dem Mischer 3, der in der dargestellten Ausführungsform ein dynamischer, das heißt angetriebener, Mischer ist, verbindbar. Hierzu werden Einlassstutzen 3a, 3b des Mischers in die Auslassstutzen 6, 7 eingeschoben. Wie auch aus der Detailansicht der Figur 5b ersichtlich ist, ist an dem Mischer 3 eine Führungsrippe 13 ausgebildet, die von dem hinteren Ende des Mischers 3 in Richtung zu der Doppelkartusche 1 wegragt. Auf jedem der Auslassstutzen 6, 7 ist eine Rastnocke 14 vorgesehen, die gemeinsam einen Führungskanal 14b bilden, dessen Breite derart bemessen ist, dass die Führungsrippe 13 des Mischers 3 zwischen den beiden Rastnocken 14 geführt wird, wenn der Mischer 3 auf die Auslassstutzen 6, 7 aufgesteckt wird. In der dargestellten Ausführungsform ist zusätzlich auf jedem Auslassstutzen 6, 7 ein Führungssteg 14a vorgesehen, der sich in axialer Richtung erstreckt und mit der Führungsrippe 13 des Mischers 3 zusammenwirkt. Die Rastnocken 14 und Führungsstege 14a, die gemeinsam den Führungskanal 14b definieren, erleichtern somit in Zusammenwirken mit der Führungsrippe 13 das positionsgenaue Aufsetzen des Mischers 3. Das Einführen einer in den Figuren nicht dargestellten Mischerwelle eines Ausbringgerätes in eine entsprechende Aufnahme des Mischers 3 kann dadurch erleichtert werden, dass in der Führungsrippe 13 des Mischers 3 eine Aussparung 13a vorgesehen ist, die den Blick auf die bspw. mit einem Innensechskant gestaltete Aufnahme des Mischers 3 freigibt.

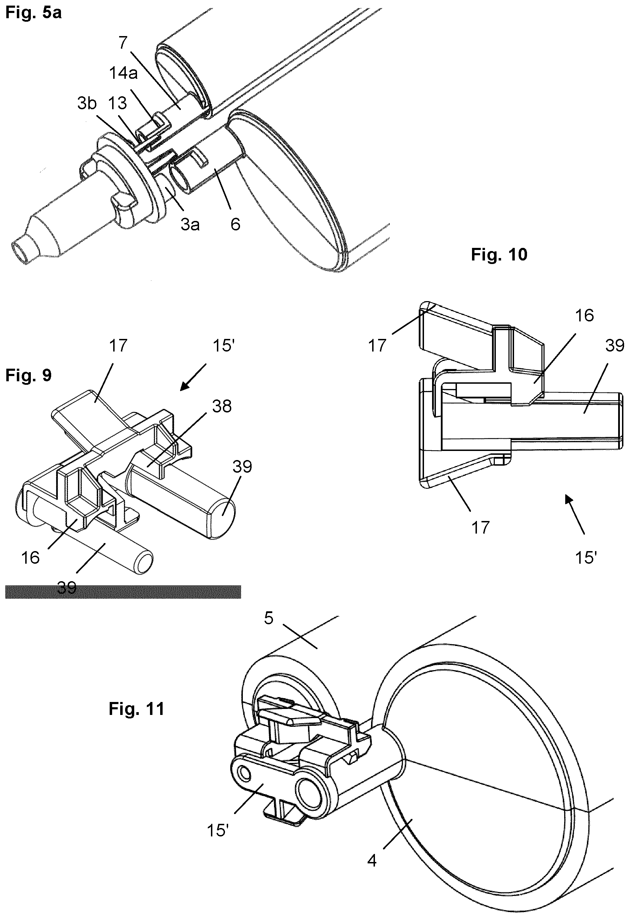

Aus der Darstellung der Figur 5a geht hervor, dass die Führungsrippe 13 des Mischers 3 beim Aufsetzen eines Mischers in dem Führungskanal 14b geführt wird, noch bevor die Auslassstutzen 6, 7 mit den Einlassstutzen 3a, 3b in Kontakt treten. Damit wird das Einstecken der Einlassstutzen 3a, 3b in die Auslassstutzen 6, 7 erleichtert. Darüber hinaus kann auf der in Figur 5a unteren Seite der Auslassstutzen 6, 7 eine Barriere bzw. ein Anschlag (nicht dargestellt) vorgesehen sein, die verhindern, dass der Mischer um 180° gedreht aufgesetzt wird. Mit anderen Worten können eine solche Barriere oder ein Anschlag verhindern, dass der Einlassstutzen 3a mit dem Auslassstutzen 7 in Kontakt tritt bzw. dass der Einlassstutzen 3b mit dem Auslassstutzen 6 in Kontakt tritt. Hierzu sind die Barriere bzw. der Anschlag vorzugsweise so angeordnet, dass die Führungsrippe 13 des Mischers 3 gegen diese stoßen würde, bevor sich die Einlassstutzen und die Auslassstutzen berühren.

Eine weitere Funktion der Rastnocken 14 ist aus Figur 4 ersichtlich, in welcher auf das vordere Ende der Doppelkartusche 1 ein Verschlussstopfen 15 aufgesetzt ist. Dieser weist eine Platte bzw. einen Steg auf, von welchem zwei Stifte in Richtung der Doppelkartusche 1 wegragen, die dichtend in die Auslassstutzen 6, 7 einsteckbar sind. Der Verschlussstopfen 15 weist weiter Rasthaken 16 auf, welche die Rastnocken 14 auf den Auslassstutzen 6, 7 hintergreifen, wenn der Verschlussstopfen 15 die beiden Auslassstutzen verschließt. Zum Lösen der Verrastung ist eine Betätigungslasche 17 vorgesehen, welche in Ausbringrichtung, das heißt in Figur 4 nach rechts gezogen werden kann, um zunächst die Verrastung zu lösen und dann den Verschlussstopfen 15 von den Auslassstutzen 6, 7 abzuziehen. Da der Verschlussstopfen 15 beide Auslassstutzen 6, 7 gleichzeitig verschließt, dient der Verschlussstopfen 15 auch zur Aussteifung bzw. Stabilisierung der Doppelkartusche 1, deren Vorratsbehälter 4, 5 im Übrigen ausschließlich über die Brücke 9 miteinander verbunden sind.

Die Stützkartusche 2 ist in der dargestellten Ausführungsform aus zwei einstückig miteinander verbundenen Metallrohren gebildet, welche beidseits offen sind. Die Rohre, die bspw. aus Aluminium bestehen, haben eine Wandstärke von etwa 1 mm bis etwa 2 mm, insbesondere etwa 1,3 mm. An dem in Figur 1 linken, vorderen Ende sind diese Rohre der Stützkartusche 2 zumindest bereichsweise mit einem nach innen ragenden Kragen 18 versehen, der mit der Nut 11 der Doppelkartusche 1 zusammenwirken kann, um die Doppelkartusche 1 in der Stützkartusche 2 abzustützen. Darüber hinaus ist die Länge der Doppelkartusche 1 derart an die Länge der Stützkartusche 2 angepasst, dass die Brücke 9 bzw. der flanschartige Rand 10 am hinteren Ende der Doppelkartusche 1 an dem hinteren Ende des jeweiligen Rohres der Stützkartusche 2 anliegt, wenn die Doppelkartusche 1 in die Stützkartusche 2 eingebracht wird. Damit ist die Doppelkartusche 1 in Vorschubrichtung der Ausbringkolben 8 an ihren beiden Enden in der Stützkartusche 2 abgestützt und gesichert.

Alternativ zu der dargestellten Ausführungsform kann die Stützkartusche 2 mit einer stirnseitigen Wand versehen sein, die den nach innen ragenden Kragen 18 ersetzt oder sich an diesem abstützt. Eine solche stirnseitige Wand oder Platte kann auch in die Stützkartusche 2 eingeschraubt oder eingeklebt sein. Durch eine stirnseitige Wand wird die Doppelkartusche 1 noch besser in der Stützkartusche 2 abgestützt. Die Wand kann entweder Öffnungen aufweisen, die das Durchtreten der Auslassstutzen 6, 7 ermöglichen oder es können in der Wand selbst Stutzen vorgesehen sein, die die Auslassstutzen 6, 7 aufnehmen können.

Zusätzlich ist in jedem Rohr der Stützkartusche 2 ein Fenster 19 vorgesehen, durch welches die Doppelkartusche 1 von außen sichtbar ist. Dies ermöglicht es auch, bspw. eine Farbmarkierung oder dgl. Kodierung auf der Doppelkartusche 1 durch das Fenster 19 zu erkennen.

In der Stützkartusche 2 ist in einem Bereich zwischen den beiden Rohren ein Schlitz zur Aufnahme einer Adapterschiene 20 vorgesehen. Die Adapterschiene 20 kann in diesen Schlitz eingeschoben und dort mittels einer Schraube 21 fixiert werden. Die Adapterschiene 20 kann wie die Stützkartusche 2 aus Metall, insbesondere Aluminium, bestehen oder vorzugsweise aus Kunststoff.

Weiter kann die Stützkartusche 2 mit einem Sicherungsbügel 22 versehen sein, welcher an einem Scharnier 23 in der Adapterschiene 20 schwenkbar angelenkt werden kann. Der Sicherungsbügel 22 weist einen etwa U-förmigen Haltebereich 24 auf, welcher den Mischer 3 bereichsweise umgreifen und damit auf den Auslassstutzen 6, 7 befestigen kann. Über einen Rasthaken 25 kann der Sicherungsbügel 22 in der Adapterschiene 20 bzw. der Stützkartusche 2 in seiner den Mischer 3 fixierenden Position befestigt werden. Um den Sicherungsbügel 22 aus seine sich etwa parallel zu den Rohren der Stützkartusche 2 erstreckenden, verrasteten Position (Figur 2) in einer den Mischer 3 freigebende Position (Figur 3) zu verschwenken, ist ein Entriegelungsknopf 26 vorgesehen, der den Rasthaken 25 in eine die für Rastung freigebende Stellung verschwenken kann. Durch eine in Figur 1 angedeutete Feder 27 kann der Sicherungsbügel 22 nach Betätigung des Entriegelungsknopfes 26 selbsttätig in die in Figur 2 gezeigte Position verschwenken, in welcher ein Wechsel bzw. die Montage des Mischers 3 möglich ist. Weiter ist in der Adapterschiene 20 bzw. der Stützkartusche 2 ein Rastvorsprung vorgesehen, welchen der Rasthebel 12 der Doppelkartusche 1 hintergreift, wenn diese in die Stützkartusche 2 eingeschoben wird. Die Doppelkartusche 1 ist hierdurch auch gegen die Vorschubrichtung der Ausbringkolben 8 innerhalb der Stützkartusche 2 gesichert. Zur Entnahme der Doppelkartusche 1 aus der Stützkartusche 2 muss ein Benutzer auf den oberen Bereich des Rasthebels 12 drücken, um die Verrastung wieder zu lösen.

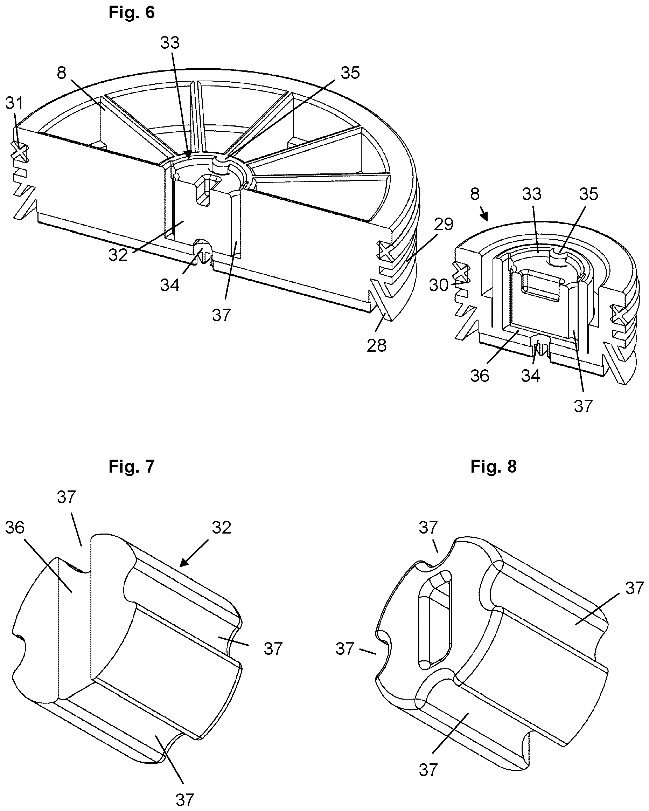

Wie in Figur 6 dargestellt, sind die Ausbringkolben jeweils mit zwei angeformten Dichtlippen 28 sowie mit zwei Abstreiferstegen 29 versehen, die um den Außenumfang der Ausbringkolben umlaufen. Zusätzlich ist in einer umlaufenden Nut 30 ein Dichtungsring, der bspw. als O-Ring oder als X-Ring ausgebildet sein kann, aufgenommen. In jedem Ausbringkolben 8 verläuft ein Entlüftungskanal, welcher über einen Drehverschluss 32 abgedichtet werden kann. Hierzu ist in jedem Ausbringkolben eine im Wesentlichen zylindrische Aufnahme für einen Drehverschluss 32 vorgesehen. Diese Aufnahme weist an ihrem in Figur 6 oberen Rand eine Wulst 33 auf, die eine Bewegung des Drehverschlusses 32 in axialer Richtung verhindert. Auf diese Weise wird bei sehr hohen Austragkräften des Ausbringgerätes (Dispensers) und dem damit verbundenen Gegendruck der Dentalmasse verhindert, dass der Drehverschluss 32 axial aus dem Ausbringkolben wieder nach hinter herausgedrückt wird. Dies würde sonst zu einer Undichtigkeit und damit Verschmutzung des Ausbringgerätes führen. Alternativ zu dem Wulst 33 kann auch eine Gewinde- oder Bajonettverbindung zwischen der Aufnahme und dem Drehverschluss 32 vorgesehen sein.

Die zylindrische Aufnahme steht über eine Entlüftungsöffnung 34 mit dem Innenraum der Vorratsbehälter 4, 5 in Verbindung. Weiter sind in dem dargestellten Ausführungsbeispiel an zwei einander gegenüberliegenden Seiten Rastvorsprünge 35 auf der Innenseite der zylindrischen Aufnahme vorgesehen.

Die in den Figuren 7 und 8 im Detail dargestellten Drehverschlüsse 33 sind jeweils mit einer Bodennut 36 in der in Figur 6 unteren Seite sowie vier in axialer Richtung verlaufenden seitlichen Nuten 37 versehen, von denen jeweils zwei mit der Bodennut 36 verbunden sind, während die beiden übrigen seitlichen Nuten 37 nicht mit der Bodennut 36 verbunden sind. Auf der der Bodennut 36 gegenüberliegenden Seite ist in jedem Drehverschluss 32 bspw. ein Schlitz vorgesehen, um den Drehverschluss mittels eines Werkzeugs in der zylindrischen Aufnahme zu drehen. Die Rastvorsprünge 35 auf der Innenseite der zylindrischen Aufnahme verhindern dabei eine unbeabsichtigte Drehung der Drehverschlüsse 32.

In dem in Figur 6 rechten Ausbringkolben nehmen die beiden nicht mit der Bodennut 36 verbundenen seitlichen Nuten 37 die Rastvorsprünge 35 auf, so dass über die beiden mit der Bodennut 36 verbundenen seitlichen Nuten 37 ein über die Entlüftungsöffnung 34 mit dem Innenraum der Vorratsbehälter 4, 5 verbundener Entlüftungskanal gebildet wird. Demgegenüber ist in dem in Figur 6 linken Ausbringkolben der Drehverschluss 32 um 90° gedreht, so dass die beiden mit der Bodennut 36 verbundenen seitlichen Nuten 37 durch die Rastvorsprünge 35 verschlossen werden. Auf diese Weise ist es möglich, nach dem Befüllen der Vorratsbehälter 4, 5 und dem Einsetzen der Ausbringkolben 8 zunächst Restluft aus den Vorratsbehältern entweichen zu lassen und die Ausbringkolben danach durch Betätigung der Drehverschlüsse 32 vollständig abzudichten.

Die Figuren 9 bis 11 zeigen eine weitere Ausführungsform eines Verschlussstopfens 15', bei welchem die Betätigungslaschen 17 so in Richtung zu der Kartusche versetzt sind, dass die Betätigungslaschen 17 im Wesentlichen nicht über das der Kartusche abgewandte Ende des Verschlussstopfens 15' hervorstehen. Dies vermindert die Gefahr von Beschädigungen während des Transports und verringert den erforderlichen Stauraum in Umverpackungen. Weiter sind Versteifungselemente 38 vorgesehen, die die Führungssteg 14a so umgreifen bzw. aufnehmen, dass die Verwindungssteifigkeit der Doppelkartusche bei aufgesetztem Verschlussstopfen 15' verbessert wird. Weiter sind in den Figuren 9 und 10 die in die Auslassstutzen eingreifenden Vorsprünge 39 zu erkennen, die die Behälter der Doppelkartusche bei aufgesetztem Verschlussstopfen 15' verschließen.

Der Mischer 3 ist in den dargestellten Ausführungsformen auf das Kartuschensystem aufsteckbar und kann über den Sicherungsbügel 22 befestigt werden. Zusätzlich oder alternativ hierzu kann der Mischer auch mit einem Verriegelungssystem versehen sein, das ähnlich der Befestigung der Verschlussstopfen 15 bzw. 15' ausgebildet ist. Mit anderen Worten kann auch der Mischer 3 zur lösbaren Befestigung an einem Kartuschensystem bspw. mit Rasthaken 16 und einer Betätigungslasche 17 versehen sein.

In den Figuren ist die Doppelkartusche als eine einstückige Verpackung dargestellt. Alternativ können auch miteinander verbundene oder miteinander verbindbare einzelnen Behälter, wie Schlauchbeutel, die Doppelkartusche bilden.

Nach einer weiter bevorzugten Ausführungsform beträgt die Wandstärke der Vorratsbehälter 4 bzw. 5 weniger als ein Zehntel der Dicke der Brücke 9, bspw. etwa 0,5 mm bis etwa 1,0 mm, vorzugsweise etwa 0,7 mm oder etwa 0,9 mm. Die Stirnwand kann dagegen eine Dicke von etwa 7 mm aufweisen, um größere Kräfte aufnehmen zu können.

Weiter wird es bevorzugt, wenn in der Innenseite der Stirnwand, insbesondere im Übergangsbereich zwischen der Stirnwand und der Seitenwand, eine umlaufende Nut ausgebildet ist. Hierdurch lässt sich die erfindungsgemäße Kartusche 1 auch dann weitestgehend entleeren, wenn (Ausbring-)Kolben 8 mit einer radial äußeren, in Vorschubrichtung weisenden Dichtlippe 28 eingesetzt werden. Die Dichtlippe 28 kann dabei in die Nut eintauchen. Die Nut bietet zudem Vorteile bei der Spritzgusstechnik im Übergang zwischen der dünneren Seitenwand und der stärkeren Stirnwand.

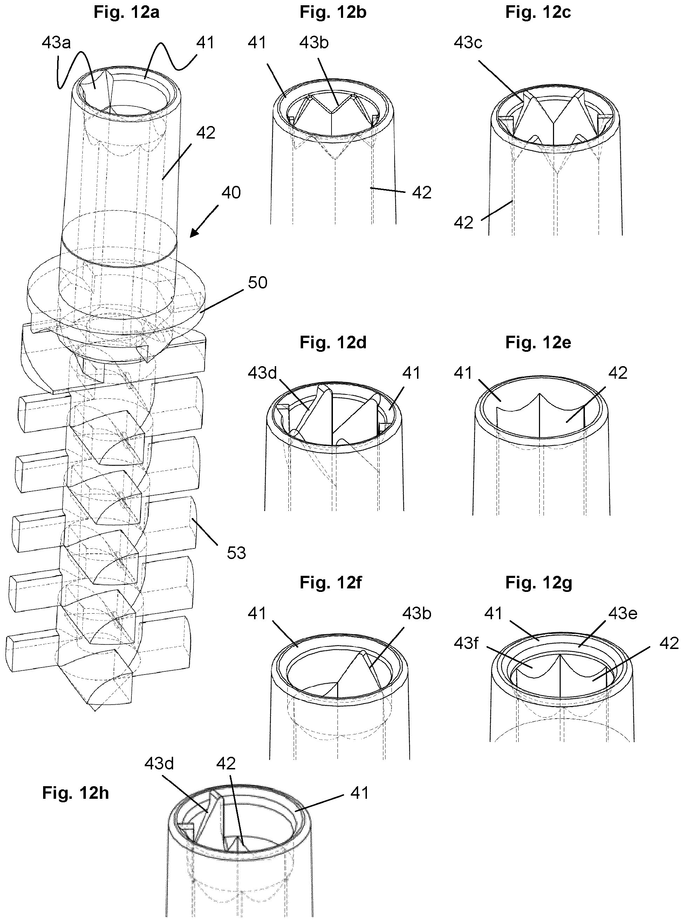

In den Figuren 12a bis 12h sind verschiedene Ausgestaltungen des Aufnahmebereichs einer Mischerwelle 40 zur Aufnahme einer Antriebswelle (nicht dargestellt) gezeigt. Die dargestellten Innenkonturen der Aufnahmebereiche sollen jeweils das Einführen der mit einem Außensechskant versehenen Antriebswelle erleichtern, insbesondere wenn der Außensechskant der Antriebswelle nicht ideal passend zu der entsprechenden Gegenkontur des Aufnahmebereichs ausgerichtet ist, während die Antriebswelle in die Mischerwelle 40 eingeführt wird.

Hierzu ist in Figur 12a die Mischerwelle 40 des dynamischen (antreibbaren) Mischers 3 mit einer ringförmigen (konischen) Phase 41 dargestellt. Die Mischerwelle 40 ist zur drehfesten Aufnahme einer Antriebswelle (nicht dargestellt) mit einem Innensechskant 42 ausgestattet, wobei eine Rame 43a vorgesehen ist, die das Einführen der mit einem Außensechskant versehenen Antriebswelle erleichtert. Die Rampe 43a ist nach innen geneigt, so dass falls eine Kante des Außensechskants der Antriebswelle auf diese Rampe 43a trifft, die Antriebswelle durch fortgesetztes Einführen der Antriebswelle in die Mischerwelle 40 soweit gedreht wird, bis eine Fläche des Außensechskants der Antriebswelle an der Rampe 43a anliegt, d.h. bis der Außensechskant passend zu dem Innensechskant 42 ausgerichtet ist.

Statt der Rampe 43a sind in der Ausführungsform nach Figur 12b zwischen der ringförmigen (konischen) Phase 41 und dem Innensechskant 42 Dreiecke 43b vorgesehen, die das Einführen der mit einem Außensechskant versehenen Antriebswelle erleichtern. Durch die in Figur 12b nach oben weisenden Schrägflächen der Dreiecke 43 kann bei einer etwaige Fehlstellung des Außensechskants dieser durch fortgesetztes Einführen der Antriebswelle in die Mischerwelle 40 soweit gedreht werden, bis der Außensechskant passend zu dem Innensechskant 42 ausgerichtet ist.

Eine ähnliche Ausführungsform ist in Figur 12c gezeigt, wobei statt der Dreiecke 43b an mehreren um den Umfang verteilten Stellen jeweils ein keilförmiger Einlauf 43c vorgesehen ist. In Figur 12d sind einseitige Einlaufschrägen 43d vorgesehen, die eine Drehung des Außensechskants nur in eine Richtung erlauben. In Figur 12e ist die konische Phase 41 gegenüber den zuvor genannten Ausführungsformen so weit verlängert, dass diese eine direkt in den Innensechskant 42 einlaufende Schräge ausbildet.

Die Ausführungsform der Figur 12f entspricht weitgehend der der Figur 12b, wobei nur ein einziges Dreieck 43b vorgesehen ist. In gleicher Weise entspricht die Ausführungsform der Figur 12h entspricht weitgehend der der Figur 12d, wobei nur ein zwei sägezahnartige Spitzen 43d vorgesehen sind, die allerdings einander mit ihren schrägen Flächen zugewandt sind.

Die Ausführungsform nach Figur 12g basiert auf der Ausführungsform der Figur 12e, wobei im Anschluss an die konische Phase 41 zunächst ein zylindrischer Führungsbereich 43e sowie daran anschließend ein weiterer konischer Bereich 43f vorgesehen ist, der dann in den Innensechskant 42 übergeht.

Hierbei zentriert und führt die der ringförmige Phase 41 zunächst die Antriebswelle. Die Dreiecke 43 führen dann bei stehendem Mischer die Kanten des Außensechskant der Antriebswelle in die Innensechskant-Kontur 42 der Mischerwelle 40, so dass diese sich maximal um 1/12 drehen muss, um den Außensechskant aufzunehmen.

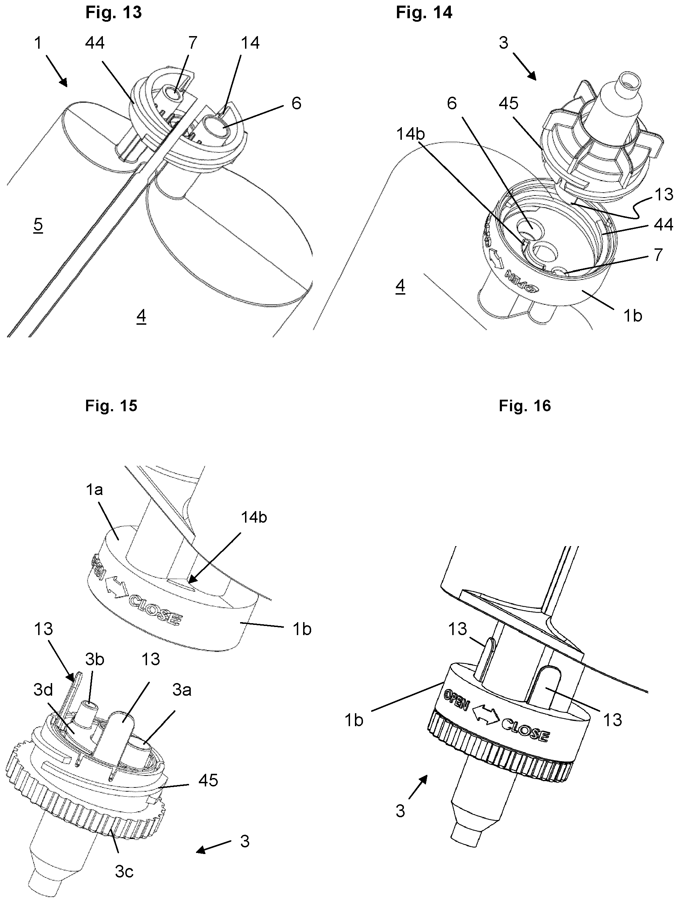

Abweichend von den zuvor beschriebenen Ausführungsformen kann die Doppelkartusche 1 auch mit einem Gewinde 44 versehen sein, das zur Befestigung des Mischers 3 dient. In Figur 13 ist dabei eine Doppelkartusche dargestellt, bei welcher die an den Auslassstutzen 6, 7 vorgesehenen Vorsprünge 14 einstückig mit einem Ring bzw. Ringsegmenten ausgebildet sind, der beide Auslassstutzen umgibt. Dieser Ring bzw. die Ringsegmente tragen ein Außengewinde 44, auf welchem bspw. mittels einer Überwurfmutter (nicht dargestellt) ein Mischer 3 befestigt werden kann. Eine Führungsrippe 13 eines ebenfalls nicht gezeigten Mischers kann dabei zwischen den Vorsprüngen 14 so geführt werden, dass der Mischer zu der Doppelkartusche so ausgerichtet wird, dass die Einlassstutzen des Mischers die Auslassstutzen der Kartusche treffen.

Eine ähnliche Ausführungsform ist in Figur 14 gezeigt. Hierbei ist der Ring bzw. Kragen 1b am Ende der Auslassstutzen 6, 7 mit einem Innengewinde 44 versehen, in welches ein entsprechendes Außengewinde 45 auf dem Gehäuse des Mischers 3 eingeschraubt werden kann. Hierzu ist ein innerer Einsatz des Mischers, der auch die Einlassstutzen 3a, 3b des Mischers aufweist, drehbar in dem Gehäuse des Mischers 3 aufgenommen, so dass das äußere Gehäuse zum Aufschrauben des Mischers relativ zu dem inneren Einsatz verdreht werden kann. Der das Innengewinde 44 tragende Kragen 1b kann einstückig mit einer Platte 1a ausgebildet sein, in die die Auslassstutzen bündig münden. Die Vorsprünge 14 der beiden Auslassstutzen 6, 7 bilden somit gemeinsam die Platte 1a, wobei die Führungsrippe 13 des Mischers in eine Öffnung bzw. einen Führungskanal 14b der Platte eingreift, um den Mischer bzw. dessen inneren Einsatz so zu der Doppelkartusche auszurichten, dass die Einlassstutzen des Mischers die Auslassstutzen der Kartusche treffen. Wie aus Figur 14 ersichtlich ist, ist die Breite der Führungsrippe 13 größer als der Durchmesser des größeren Auslassstutzens 6, so dass die Führungsrippe 13 nicht versehentlich in einen der Auslassstutzen gesteckt werden kann.

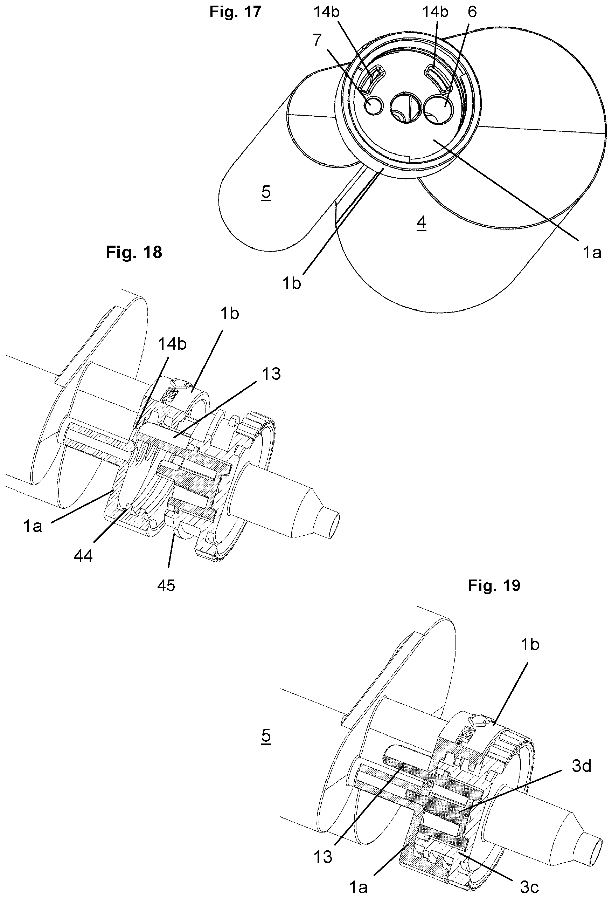

Die Figuren 15 bis 19 zeigen eine der Figur 14 ähnliche Ausführungsform, bei welcher statt einer Führungsrippe 13 zwei voneinander beabstandete Führungsrippen 13 vorgesehen sind, die jeweils in eine Öffnung bzw. einen Führungskanal 14b der Platte 1a eingesteckt werden können. Die Spitzen der Führungsrippen 13 können dabei auf der dem Mischer 3 abgewandten Seite aus der Platte 1a der Kartusche vorstehen, wenn der Mischer vollständig auf der Kartusche befestigt ist. Hierbei kann auch eine Farbmarkierung verwendet werden, um einem Benutzer die Befestigung des Mischers anzuzeigen. Wie aus den Schnittansichten der Figuren 18 und 19 hervorgeht, ist unabhängig von den zuvor beschriebenen Merkmalen ein erfindungsgemäßer Mischer 3 vorzugsweise mit einem Mischergehäuse 3c, das das Außengewinde 45 trägt, und einem Ankopplungsabschnitt 3d ausgebildet, der frei drehbar, d.h. in beide Richtungen willkürlich drehbar, in dem Mischergehäuse 3c gelagert ist. Der Ankopplungsabschnitt 3d weist hierbei die wenigstens eine Führungsrippe 13 sowie die Einlassstutzen 3a und 3b auf und schließt das Mischergehäuse 3c kartuschenseitig ab. Zusätzlich kann in dem Ankopplungsabschnitt 3d die Mischerwelle 40 gelagert sein. Die freie Drehbarkeit des Mischergehäuses relativ zu dem Ankopplungsabschnitt bietet gegenüber bekannten Lösungen den Vorteil, dass der Mischer in beliebiger Position auf die Kartusche aufgesetzt werden kann und sich die Kartusche und der Ankopplungsabschnitt frei zueinander ausrichten können, ohne dass hierfür ein Benutzer direkten Sichtkontakt bspw. zu den Einlassstutzen und/oder der Führungsrippe haben muss. Zudem wird hierdurch erst das Einschrauben des Mischers möglich, während die Ein- und Auslassstutzen sowie die Führungsrippe und der Führungskanal bereits ineinander eingreifen.

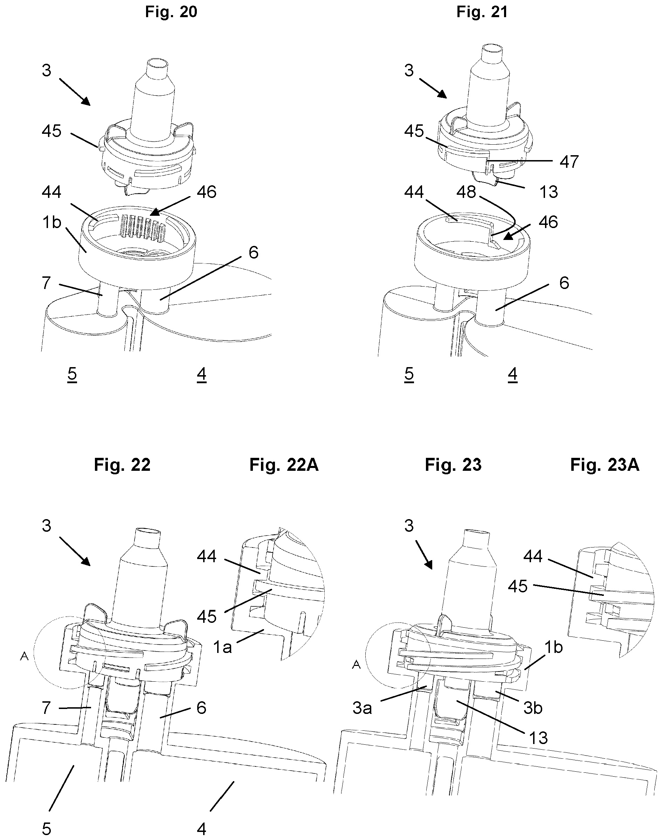

Die Ausführungsform nach Figur 20 zeigt eine ähnliche Ausgestaltung des Mischers 3 mit einem Außengewinde 45, wobei sich die das Gewinde 45 bildenden Vorsprünge oder Stege nur um einen Teil des Umfangs des Mischers erstrecken und die einzelnen Gewindestegsegmente einander nicht überlappen. Vielmehr verbleiben zwischen den einzelnen Gewindesegmenten Freiräume. Entsprechend sind auch für das Innengewinde 44 der Kartusche nur einzelne einander nicht überlappende Gewindestegsegmente mit dazwischen angeordneten Freiräumen vorgesehen. Die Größe der Freiräume ist dabei so gewählt, dass die Gewindesegmente des Außengewindes in die Freiräume zwischen den Segmenten des Innengewindes passen und umgekehrt.

Während des Einschraubens des Mischers in das Innengewinde 44 der Kartusche liegen die in Figur 20 oberen Flächen der Gewindestegsegmente des Außengewindes 45 an den in Figur 20 unteren Flächen der Gewindestegsegmente des Innengewindes 44 an, um den Mischer 3 in den Ring 1b der Kartusche zu ziehen.

Die Gewinde 44, 45 sind dabei so ausgelegt, dass diese beim Aus- bzw. Abschrauben des Mischers 3 außer Eingriff treten, da sich die die jeweiligen Gewindesegmente des Innengewindes 44 hintergreifenden Segmente des Außengewindes 45 von der jeweiligen unteren Fläche der Gewindestegsegmente des Innengewindes 44 entfernen. Mit anderen Worten kann der Mischer 3 durch ein Lösen des Gewindeeingriffs zunächst nicht von der Kartusche bzw. der Platte 1a, in die die Auslassstutzen 6, 7 münden, abgehoben werden.

Dies ist jedoch in einigen Fällen wünschenswert, insbesondere wenn der Mischer durch aushärtendes Gemische an der Kartusche festklebt. Daher ist auf der Innenwand des Rings 1b, der die Platte 1a der Kartusche umgibt, eine Rampe 46 ausgebildet, die aus den Kopfflächen mehrerer axial verlaufender Stege definiert wird. Nachdem das Mischergehäuse durch eine Drehung relativ zu der Kartusche so weit abgeschraubt ist, dass sich die Gewindesegmente der beiden Gewinde nicht mehr überdecken, treten die in Figur 20 unteren Flächen der Gewindestegsegmente des Außengewindes 45 mit den Kopfflächen der axialen Stege, d.h. mit der Rampe 46, in Kontakt, um den Mischer 3 von der Platte 1a der Kartusche abzuheben, in die die Auslassstutzen 6, 7 münden.

Figur 21 zeigt eine Abwandlung der Ausführungsform der Figur 20, wobei die Gewindesegmente des Innengewindes 44 und des Außengewindes 45 wiederum mit entsprechenden Freiräumen gestaltet sind. Das Einschrauben des Mischers 3 in den Ring der Kartusche erfolgt somit wie oben unter Bezug auf Figur 20 erläutert. Gleiches gilt für das Lösen der Gewindeverbindung, wobei wiederum die Gewindesegmente zunächst außer Eingriff kommen, ohne dass der Mischer von der Platte der Kartusche abzuheben, in die die Auslassstutzen 6, 7 münden.

Bei der Ausführungsform nach Figur 21 sind im Übergangsbereich zwischen dem Ring 1b der Kartusche und der Platte 1a, in die die Auslassstutzen 6, 7 münden, Rampen 46 vorgesehen, die jeweils über einen axial verlaufenden Steg an der Innenwand des Rings 1b mit dem entsprechenden Gewindesegment des Innengewindes 44 verbunden sein können. Von den Enden der Gewindesegmente des Außengewindes 45 ragen auf der Außenfläche des Mischergehäuses axiale Stege 47 in Richtung der Kartusche. Die kartuschenseitigen Enden (unten in Figur 21) dieser Stege 47 reichen bis zu dem kartuschenseitigen Rand des Mischergehäuses bzw. können geringfügig darüber hinausstehen. Die Rampen 46 und Stege 47 bewirken, dass nachdem das Mischergehäuse durch eine Drehung relativ zu der Kartusche so weit abgeschraubt ist, dass sich die Gewindesegmente der beiden Gewinde nicht mehr überdecken, bei fortgesetzter Drehung des Mischers die kartuschenseitigen Enden der Stege 47 mit den Rampen 46 in Kontakt treten, um den Mischer 3 von der Platte 1a der Kartusche abzuheben. Zusätzlich kann auch der Ring (Kragen) 1b der Kartusche mit axialen Stegen 48 versehen sein, die mit den Stegen 47 des Mischers 3 so zusammenwirken, dass eine Drehung des Mischers entgegen der Einschraubrichtung der Gewinde 44, 45 nur so weit möglich ist, bis der Mischer 3 von der Kartusche abgenommen werden kann bzw. durch die Rampe 46 abgehoben wird.

Eine weitere Ausführungsform ist in den Figuren 22 bis 23A dargestellt. Der grundsätzliche Aufbau der Kartusche und des Mischers entspricht dabei im Wesentlichen der in Figur 14 gezeigten Ausführungsform. Im Unterschied hierzu sind die Gewinde 44, 45 auf dem Mischergehäuse bzw. in dem Ring 1b der Kartusche jedoch so ausgebildet, dass die Gewindegänge, d.h. die Freiräume zwischen den von der Innenwand des Rings 1b bzw. von der Außenwand des Mischergehäuses vorspringenden Gewindesegmenten breiter gewählt sind als die Breite der Gewindesegmente. Beispielsweise können die Gewindegänge eine Breite von etwa 2,3 mm haben, während die Gewindesegmente nur eine Breite von 1,55 mm aufweisen. Hierdurch entsteht zwischen den einzelnen Gewindesegmenten ein Spiel bzw. ein Freiraum, so dass beim Einschrauben des Mischers 3 in den Ring 1b der Kartusche (Figuren 22 und 22A) nur die in den Figuren oberen Flächen der Gewindestegsegmente des Außengewindes 45 an den in den Figuren unteren Flächen der Gewindestegsegmente des Innengewindes 44 anliegen, um den Mischer 3 in den Ring 1b der Kartusche zu ziehen. Umgekehrt liegen beim Aus- oder Abschrauben des Mischers 3 (Figuren 23 und 23A) nur die in den Figuren unteren Flächen der Gewindestegsegmente des Außengewindes 45 an den in den Figuren oberen Flächen der Gewindestegsegmente des Innengewindes 44 an. Hierdurch lässt sich der Reibwiderstand erheblich reduzieren.

Zusätzlich kann der Außendurchmesser der Gewindesegmente des Außengewindes definiert kleiner als der Innendurchmesser des Rings 1b in den Gewindegängen gewählt sein und der Innendurchmesser der Gewindesegmente des Innengewindes definiert größer als der Außendurchmesser des Mischergehäuses in den Gewindegängen gewählt sein. Wie in den Figuren 22 bis 23A gezeigt, entsteht hierdurch auch im Bereich der Gewinde 44, 45 ein radiales Spiel zwischen dem Ring 1b der Kartusche und dem Mischergehäuse, so dass der Mischer 3 in der Kartusche ausschließlich über die Führungsrippe(n) 13 und den Führungskanal 14b geführt ist, bevor auch die Einlassstutzen 3a, 3b und die Auslassstutzen 6, 7 ineinander eingreifen. Durch diese Gestaltung des Gewindes werden folglich Doppelpassungen vermieden und die zum Ein- bzw. Ausschrauben erforderlichen Kräfte können weiter reduziert werden.

In der Ausführungsform der Figuren 22 bis 23A reicht das der Kartusche zugewandte Ende der Gewindesegmente 45 des Mischers 3 nicht bis zu der kartuschenseitigen Kante des Mischergehäuses. Hierdurch entsteht an dem kartuschenseitigen Ende des Mischergehäuses ein zylindrischer Abschnitt, der kein Gewinde trägt. Dieser Abschnitt kann genutzt werden, um das Mischergehäuse zusätzlich zu der Führung durch die Führungsrippe(n) 13 und den Führungskanal 14b innerhalb des Rings bzw. des flanschartigen Randes 1b der Kartusche, in welchem sich des Innengewinde 44 befindet, zu führen.

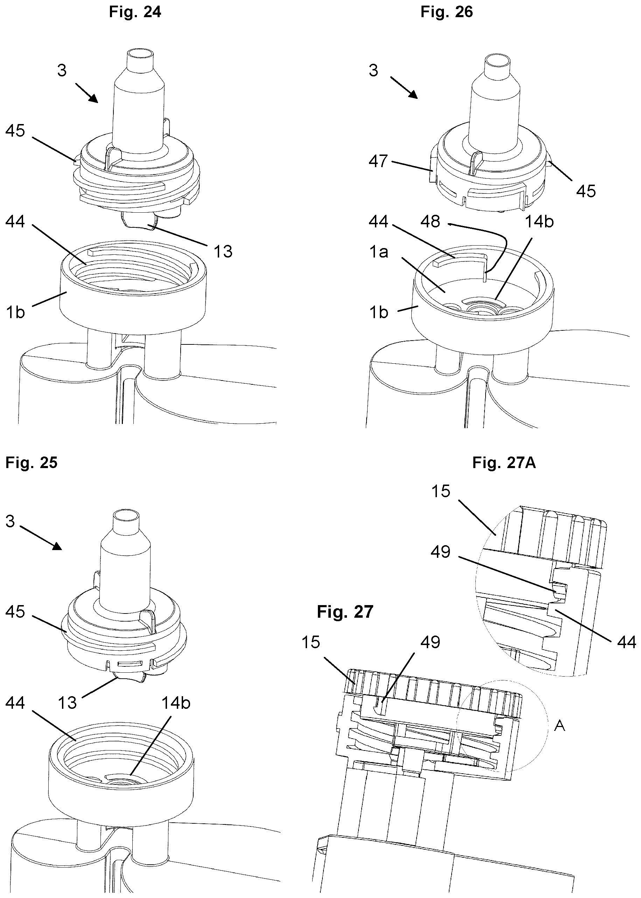

Alternativ hierzu ist es erfindungsgemäß möglich, auf eine solche zusätzliche Führung zu verzichten, so dass während des Aufsetzens des Mischers 3 auf die Doppelkartusche 1 der Mischer zunächst ausschließlich über die wenigstens eine in den Führungskanal 14b eingreifende Führungsrippe 13 geführt und ausgerichtet ist. Hierzu kann das Außengewinde 45 des Mischergehäuses bis zu der kartuschenseitigen Kante des Mischergehäuses führen. Dies ist in den Ausführungsformen der Figuren 24 und 25 dargestellt, wobei der Mischer nach Figur 24 drei sich bereichsweise überlappende Gewindestegsegmente aufweist, die sich jeweils etwa über 120° erstrecken, während der Mischer nach Figur 25 zwei sich nicht überlappende Gewindestegsegmente aufweist, die sich jeweils etwa über 180° erstrecken. Erfindungsgemäß kann auch bei den Mischern nach den Figuren 24 und 25 der Kontakt zwischen den Gewinden 44, 45 beim Ein- bzw. Ausschrauben des Mischers so wie in Bezug auf die Figuren 22 bis 23A erläutert reibungsarm erfolgen, wobei sich jeweils nur eine Fläche der Gewinde berühren.

Eine weitere Ausführungsform ist in Figur 26 dargestellt, die im Aufbau im Wesentlichen der Ausführungsform der Figur 21 entspricht. Allerdings ist in der Kartusche der Figur 26 die Rampe 46 im Übergangsbereich zwischen der Platte 1a, in die die Auslassstutzen 6, 7 münden, und dem das Innengewinde 44 tragenden Ring 1b bzw. Flansch weggelassen, so dass der Mischer 3 zwar durch eine Drehung des Mischergehäuses relativ zu der Kartusche von dieser entriegelt werden kann, der Mischer wird jedoch nicht durch fortgesetzte Drehung von der Kartusche abgehoben. Vielmehr muss der Mischer 3 nach dem Lösen der Verriegelung mit der Kartusche über die Gewinde 44, 45 von der Kartusche abgezogen werden. Auch in Figur 26 sind die weiteren axialen Stege 48 auf der Innenseite des Rings 1b dargestellt, die mit den Stegen 47 die Verdrehbarkeit des Mischers 3 relativ zu der Kartusche beim Lösen der Verbindung begrenzen.

Ein weiterer Verschlussstopfen 15 ist in den Figuren 27 und 27A gezeigt, der wiederum die Auslassstutzen 6, 7 verschließende Vorsprünge aufweist und mit einem zylindrischen Abschnitt innerhalb des Rings bzw. Kragens 1b der Kartusche aufgenommen ist. Zur Verriegelung des Verschlussstopfens 15 an der Kartusche sind Haken 49 vorgesehen, die die freien Enden der Gewindesegmentstege des Innengewindes 44 hintergreifen können. Auf diese Weise lässt sich der Verschlussstopfen 15 auf die Kartusche aufrasten. Zum Lösen dieser Verbindung wird der Verschlussstopfen 15 relativ zu der Kartusche verdreht, so dass sich die Haken 49 von den Gewindesegmentstegen lösen. Gleichzeitig kann die das kartuschenseitige frei Ende des Hakens 49 mit dem benachbarten (in Figur 27A unteren) Gewindesegmentsteg in Kontakt kommen, so dass durch die Drehung der Verschlussstopfen 15 auch von der Kartusche abgehoben wird.

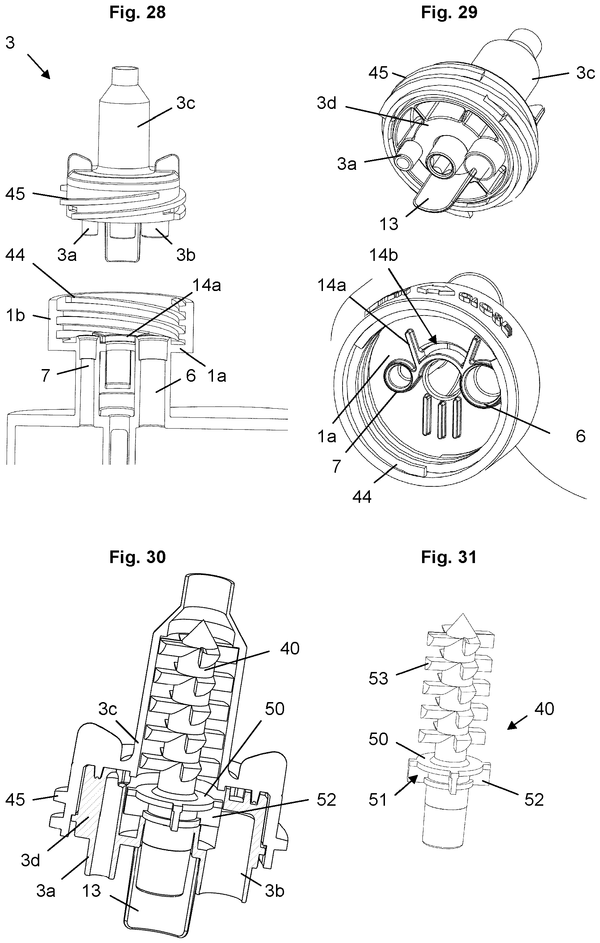

Die Figuren 28 und 29 zeigen eine weitere Ausführungsform, die im Wesentlichen der Figur 24 entspricht, d.h. die Gewinde 44, 45 sind jeweils mit einander bereichsweise überlappenden Gewindesegmentstegen ausgestaltet. Grundsätzlich können die Auslassstutzen 6, 7 einer Kartusche auch über die Platte 1a hinausragen, wie dies beispielhaft in den Figuren 28 und 29 angedeutet ist.

Unabhängig davon kann auf der Platte 1a ein Führungsvorsprung 14a ausgebildet sein, der das Einführen der Führungsrippe 13 in den Schlitz 14b erleichtert. In der in den Figuren 28 und 29 gezeigten Ausführungsform weist dieser zusätzliche Führungsvorsprung 14a zwei trichterförmig aufeinander zulaufende Bereiche auf, die beidseits des Führungsschlitzes 14b vorgesehen sind und durch einen bogenförmigen Abschnitt miteinander verbunden sind. Der zusätzliche Führungsvorsprung 14a kann dabei entweder unmittelbar mit den Auslassstutzen 6, 7 verbunden sein oder geringfügig hiervon beabstandet sein.

Zusätzlich sind in Figur 29 Rippen auf der Platte 1a zu erkennen, die dem Führungsschlitz 14b gegenüberliegen. Diese verhindern, dass der Mischer 3 in falscher Ausrichtung zu der Kartusche aufgesetzt werden kann.

In modernen Austragsystemen (Kartusche, Mischer und Ausbringgerät zum Antreiben der Kolben in der Kartusche) ist auf Grund des hohen Drucks, der während des Ausbringens entsteht, die Dichtigkeit zwischen Kartusche und Mischer besonders wichtig. Dies wird erfindungsgemäß, bspw. auch bei der Ausführungsform der Figuren 28 und 29, dadurch erreicht, dass durch ein Gewinde mit mehreren Gewindegängen eine große axiale Relativbewegung zwischen Mischer und Kartusche beim Einschrauben erzeugt wird. Hierdurch kann der Mischer fest an die Kartusche angepresst werden, so dass einerseits Ein- und Auslassstutzen dichtend miteinander verbunden werden und andererseits auch innerhalb des Mischers selbst, insbesondere zwischen dem Mischergehäuse 3c und dem Ankopplungsabschnitt 3d, die Dichtigkeit verbessert wird. Zudem nehmen die vorzugsweise um 120° versetzten und einander überlappenden Gewindegänge auch gut die auftretenden axialen Kräfte auf. Diese Gewindeverbindung erfordert jedoch, dass der Ankopplungsabschnitt und das Mischergehäuse zueinander frei drehbar sind. Dies wiederum bringt es mit sich, dass zur Vereinfachung der Ausrichtung der Ein- und Auslassstutzen beim Aufsetzen des Mischers 3 zuerst die Führungsrippe in den Führungskanal eingreift und den Mischer so führt und ausrichtet.

In den Figuren 30 und 31 ist als ein weiterer erfindungsgemäßer Aspekt eines Mischers 3 dargestellt, dass die Einlassstutzen 3a, 3b in zueinander versetzten Ebenen in den in dem Mischergehäuse 3c gebildeten Mischraum münden. Mit anderen Worten liegt der Einlass für die durch den Einlassstutzen 3b eintretende Masse näher an der Kartusche als der Einlass für die durch den Einlassstutzen 3a eintretende Masse. Hierbei wird im Inneren des Mischers ein ringförmiger Raum gebildet, der die Mischerwelle 40 umgibt, in welchem die durch den Einlassstutzen 3b eintretende Masse aufgefangen werden kann, bevor sie in den Mischraum gelangt. Dies verhindert ein zu Beginn des Mischvorgangs schlechtes Mischergebnis.

Die beiden Ebenen, in welchen die Massen eintreten, werden durch eine Scheibe 50 auf der Mischerwelle 40 voneinander getrennt, wobei Öffnungen 51 den Durchtritt der durch den Einlassstutzen 3b eintretenden Masse erlauben. Um zu erschweren, dass bei stehender Mischerwelle 40 eine Masse von einem Einlassstutzen in den anderen gelangt, sind Rippen 51 in den Zwischenräumen zwischen den Öffnungen 51 vorgesehen.

Die Mischflügel 53 der Mischerwelle und die übrige Ausgestaltung, z.B. des Verbindungsabschnitts mit einer Antriebswelle, kann wie in den Figuren 12a bis 12h gezeigt ausgebildet sein.

**Bezugszeichenliste**

| | | | |
|---|---|---|---|
| 1 | Doppelkartusche | 19 | Sichtfenster |
| 1a | Platte | 20 | Adapterschiene |
| 1b | Kragen | 21 | Schraube |
| 2 | Stützkartusche | 22 | Sicherungsbügel |
| 3 | Mischer | 23 | Scharnier |
| 3a, b | Einlassstutzen | 24 | Haltebereich |
| 3c | Mischergehäuse | 25 | Rasthaken |
| 3d | Ankopplungsabschnitt | 26 | Entriegelungsknopf |
| 4 | Vorratsbehälter | 27 | Feder |
| 5 | Vorratsbehälter | 28 | Dichtlippe |
| 6 | Auslassstutzen | 29 | Abstreifersteg |
| 7 | Auslassstutzen | 30 | Nut |
| 8 | Ausbringkolben | 31 | Dichtring |
| 9 | Brücke | 32 | Drehverschluss |
| 10 | flanschartiger Rand | 33 | Wulst |
| 11 | Nut | 34 | Entlüftungsöffnung |
| 12 | Rasthebel | 35 | Rastvorsprung |
| 13 | Führungsrippe | 36 | Bodennut |
| 13a | Aussparung | 37 | seitliche Nut |
| 14 | Rastnocke | 38 | Versteifungselement |
| 14a | Führungssteg | 39 | Vorsprung |
| 14b | Führungskanal | 40 | Mischerwelle |
| 15, 15' | Verschlussstopfen | 41 | ringförmige Phase |
| 16 | Rasthaken | 42 | Innensechskant |
| 17 | Betätigungslasche | 43a | Rampe |
| 18 | Kragen | 43b | Dreieck |
| 43c | Einlauf | 47 | Steg |
| 43d | Spitze | 48 | Steg |
| 43e | zylindrische Führung | 49 | Haken |
| 43f | ringförmige Phase | 50 | Scheibe |
| 44 | Innengewinde | 51 | Öffnung |
| 45 | Außengewinde | 52 | Rippe |
| 46 | Rampe | 53 | Mischflügel |

## Patentansprüche

1. Kombination aus einer Doppelkartusche (1) mit einem Mischer (3),
wobei die Doppelkartusche zwei Vorratsbehälter (4, 5) aufweist, die jeweils ein vorderes Ende mit einem Auslassstutzen (6, 7) aufweisen, deren Längsachsen parallel zueinander verlaufen und auf deren Außenmantelfläche jeweils wenigstens ein Vorsprung (14, 14a, 1a) angeordnet ist,
und wobei der Mischer ein Gehäuse aufweist, das einen Mischraum definiert, wobei an einem Auslassende des Gehäuses eine Auslassöffnung und an seinem gegenüberliegenden Einlassende des Mischers zwei in den Mischraum mündende Einlassstutzen (3a, 3b) vorgesehen sind, deren Längsachsen parallel zueinander verlaufen,
**dadurch gekennzeichnet, dass** der Mischer (3) ein Außengewinde (45) aufweist und die Doppelkartusche (1) zur Befestigung des Mischers (3) an der Doppelkartusche (1) ein Innengewinde (44) aufweist,
dass der wenigstens eine Vorsprung (14, 14a, 1a) derart angeordnet ist, dass die Vorsprünge gemeinsam eine Platte (1a) bilden und dass zwischen den Vorsprüngen (14, 14a; 1a) wenigstens ein Führungskanal (14b) ausgebildet wird, der durch eine Öffnung in der Platte (1a) definiert ist und der sich parallel zu den Längsachsen der Auslassstutzen (6, 7) erstreckt,
dass an dem Einlassende des Mischers (3) wenigstens eine Führungsrippe (13) vorgesehen ist, die sich parallel zu den Längsachsen der Einlassstutzen (3a, 3b) erstreckt,
und dass die Breite des zwischen den Vorsprüngen (14, 14a; 1a) gebildeten Führungskanals (14b) und die Breite der wenigstens einen Führungsrippe (13) zur Führung des Mischers (3) in der Doppelkartusche (1) aneinander angepasst sind.

2. Kombination nach Anspruch 1, **dadurch gekennzeichnet, dass** die Längen der Einlassstutzen (3a, 3b), der Führungsrippe (13) und der Auslassstutzen (6, 7) sowie die Position der Vorsprünge (14, 14a) derart aufeinander abgestimmt sind, dass beim Aufstecken des Mischers (3) auf die Doppelkartusche die Führungsrippe (13) in den zwischen den Vorsprüngen (14, 14a) gebildeten Führungskanal (14b) eingreift, bevor die Einlassstutzen (3a, 3b) die Auslassstutzen (6, 7) berühren.

3. Kombination nach Anspruch 1 oder 2, **dadurch gekennzeichnet, dass** die Breite der Führungsrippe (13) größer ist als der Öffnungsquerschnitt des größten Auslassstutzens (6) der Doppelkartusche (1).

4. Kombination nach einem der Ansprüche 1 bis 3, **dadurch gekennzeichnet, dass** auf der Platte (1a) ein Führungsvorsprung (14a) ausgebildet ist, der das Einführen der Führungsrippe (13) in den als Schlitz ausgebildeten Führungskanal (14b) erleichtert.

5. Kombination nach Anspruch 4, **dadurch gekennzeichnet, dass** der Führungsvorsprung (14a) zwei trichterförmig aufeinander zulaufende Bereiche aufweist, die beidseits des Führungskanals (14b) vorgesehen sind und durch einen bogenförmigen Abschnitt miteinander verbunden sind.

6. Kombination nach Anspruch 4 oder 5, **dadurch gekennzeichnet, dass** auf der Platte (1a) Rippen vorgesehen sind, die dem Führungskanal (14b) gegenüberliegen.

7. Mischer zur Verwendung in einer Kombination nach einem der vorhergehenden Ansprüche mit einem Gehäuse, das einen Mischraum definiert, wobei an einem Auslassende des Gehäuses eine Auslassöffnung und an seinem gegenüberliegenden Einlassende des Mischers zwei in den Mischraum mündende Einlassstutzen (3a, 3b) vorgesehen sind, deren Längsachsen parallel zueinander verlaufen, **dadurch gekennzeichnet, dass** an dem Einlassende wenigstens eine Führungsrippe (13) vorgesehen ist, die sich parallel zu den Längsachsen der Einlassstutzen (3a, 3b) erstreckt, und dass an dem Einlassende wenigstens ein als ein Außengewinde (45) ausgebildetes Verriegelungselement zur lösbaren Befestigung an einem Kartuschensystem vorgesehen ist.

8. Mischer nach Anspruch 7, **dadurch gekennzeichnet, dass** das Außengewinde (45) durch mehrere, sich nicht überlappende Gewindesegmentstege gebildet ist.

9. Mischer nach Anspruch 7 oder 8, **dadurch gekennzeichnet, dass** die Länge der Einlassstutzen (3a, 3b) in einer dem Auslassende abgewandten Richtung kleiner als die Länge der wenigstens einen Führungsrippe (13) ist.

10. Mischer nach einem der Ansprüche 7 bis 9, **dadurch gekennzeichnet, dass** die Einlassstutzen (3a, 3b) und die wenigstens eine Führungsrippe (13) Bestandteil eines Ankopplungsabschnitts (3d) des Mischers (3) sind, der frei drehbar in einem Mischergehäuse (3c) aufgenommen ist.

11. Mischer nach einem der Ansprüche 7 bis 10 mit einer antreibbaren Mischerwelle (40), die zur drehfesten Aufnahme einer Antriebswelle eine die Innensechskant-Kontur (42) aufweist, **dadurch gekennzeichnet, dass** die Mischerwelle (40) auf der Seite des Einlassendes mit einer ringförmigen Phase (41) und/oder in die Innensechskant-Kontur (42) übergehenden Dreiecken (43) versehen ist.

12. Mischer nach einem der Ansprüche 7 bis 11, **dadurch gekennzeichnet, dass** die Einlassstutzen (3a, 3b) in zueinander versetzten Ebenen in den in dem Mischergehäuse (3c) gebildeten Mischraum münden.

13. Mischer nach Anspruch 12 mit einer antreibbaren Mischerwelle (40), **dadurch gekennzeichnet, dass** die beiden Ebenen, in welchen die Massen eintreten, durch eine Scheibe (50) auf der Mischerwelle (40) voneinander getrennt sind, wobei Öffnungen (51) den Durchtritt der durch einen der Einlassstutzen (3b) eintretenden Masse erlauben.

14. Mischer nach Anspruch 13, **dadurch gekennzeichnet, dass** in den Zwischenräumen zwischen den Öffnungen (51) Rippen (52) vorgesehen sind.

15. Mischer nach einem der Ansprüche 7 bis 14 mit einer antreibbaren Mischerwelle (40), **dadurch gekennzeichnet, dass** im Inneren des Mischers (3) ein ringförmiger Raum gebildet ist, der die Mischerwelle (40) umgibt, in welchem die durch einen der Einlassstutzen (3b) eintretende Masse aufgefangen werden kann, bevor sie in den Mischraum gelangt.

## Claims

1. A combination consisting of a double cartridge (1) having a mixer (3),
wherein the double cartridge has two supply containers (4, 5), each of which has a front end with an outlet connection piece (6, 7), the longitudinal axes of which extend parallel to one another and on the outer casing surface of which in each case at least one protrusion (14, 14a, 1a) is arranged,
and wherein the mixer has a housing that defines a mixing chamber, wherein at one outlet end of the housing, an outlet opening is provided, and at its opposite inlet end of the mixer, two inlet connection pieces (3a, 3b) opening out into the mixing chamber are provided, the longitudinal axes of which extend parallel to one another,
**characterized in that** the mixer (3) has an external thread (45) and the double cartridge (1) has an internal thread (44) for fastening the mixer (3) on the double cartridge (1),
that the at least one protrusion (14, 14a, 1a) is arranged in such a manner that the protrusions jointly form a plate (1a) and that between the protrusions (14, 14a; 1a) at least one guiding channel (14b) is formed that is defined by an opening in the plate (1a) and that extends parallel to the longitudinal axes of outlet connection pieces (6, 7),
that at the inlet end of the mixer (3) at least one guiding rib (13) is provided that extends parallel to the longitudinal axes of the inlet connection pieces (3a, 3b),
and that the width of the guiding channel (14b) that is formed between the protrusions (14, 14a; 1a) and the width of the at least one guiding rib (13) for guiding the mixer (3) in the double cartridge (1) are adapted to one another.

2. The combination according to claim 1, **characterized in that** the lengths of the inlet connection pieces (3a, 3b), of the guiding rib (13) and the outlet connection pieces (6, 7) as well as the position of the protrusions (14, 14a) are aligned in such a manner that when attaching the mixer (3) onto the double cartridge (1), the guiding rib (13) engages with the guiding channel (14b) that is formed between the protrusions (14, 14a), before the inlet connection pieces (3a, 3b) make contact with the outlet connection pieces (6, 7).

3. The combination according to claim 1 or 2, **characterized in that** the width of the guiding rib (13) is larger than the cross-section of the opening of the largest outlet connection piece (6) of the double cartridge (1).

4. The combination according to any one of claims 1 to 3, **characterized in that** on the plate (1a) a guiding protrusion (14a) is formed that facilitates the insertion of the guiding rib (13) into the guiding channel (14b) that is formed as a slot.

5. The combination according to claim 4, **characterized in that** the guiding protrusion (14a) has two regions which extend towards one another in a funnel-shaped manner and which are provided on both sides of the guiding channel (14b) and are connected to one another by a curved section.

6. The combination according to claim 4 or 5, **characterized in that** ribs that face the guiding channel (14b) are provided on the plate (1a).

7. A mixer for use in a combination according to any one of the preceding claims, comprising a housing that defines a mixing chamber, wherein at one outlet end of the housing, an outlet opening is provided and at its opposite inlet end of the mixer, two inlet connection pieces (3a, 3b) opening out into the mixing chamber are provided, the longitudinal axes of which extend parallel to one another, **characterized in that** at the inlet end, at least one guiding rib (13) is provided that extends parallel to the longitudinal axes of the inlet connection pieces (3a, 3b), and that at the inlet end at least one locking element is provided that is formed as an external thread (45) for detachable fastening to a cartridge system.

8. The mixer according to claim 7, **characterized in that** the external thread (45) is formed by a plurality of non-overlapping threaded bar segments.

9. The mixer according to claim 7 or 8, **characterized in that** the length of the inlet connection pieces (3a, 3b) is shorter in a direction facing away from the outlet end than the length of the at least one guiding rib (13) .

10. The mixer according to any one of claims 7 to 9, **characterized in that** the inlet connection pieces (3a, 3b) and the at least one guiding rib (13) are integral part of a coupling section (3d) of the mixer (3) that is accommodated freely rotatable in a mixer housing (3c) .

11. The mixer according to any one of claims 7 to 10, comprising a drivable mixer spindle (40) that has an internal hexagon contour (42) for the torque-proof accommodation of a drive shaft, **characterized in that** the mixer spindle (40) is provided on the side of the inlet end with an annular chamfer (41) and/or triangles (43) transitioning into the internal hexagon contour (42).

12. The mixer according to any one of claims 7 to 11, **characterized in that** the inlet connection pieces (3a, 3b), in planes that are offset to one another, open out into the mixing chamber that is formed in the mixer housing (3c).

13. The mixer according to claim 12, comprising a drivable mixer spindle (40), **characterized in that** the two planes at which the masses enter are separated by a disk (50) on the mixer spindle (40), wherein openings (51) allow the passage of the mass entering through one of the inlet connection pieces (3b).

14. The mixer according to claim 13, **characterized in that** ribs (52) are provided in the spaces between the openings (51).

15. The mixer according to any one of claims 7 to 14, comprising a drivable mixer spindle (40), **characterized in that** an annular space is formed in the interior of mixer (3) which space surrounds the mixer spindle (40) and in which the mass entering through one of the inlet connection pieces (3b) can be captured before it reaches into the mixing chamber.

## Revendications

1. Combinaison d'une cartouche double (1) et d'un mélangeur (3),
dans laquelle la cartouche double comporte deux récipients de stockage (4, 5) comportant respectivement une extrémité avant avec un embout d'évacuation (6, 7) dont les axes longitudinaux s'étendent parallèlement l'un à l'autre et sur la surface d'enveloppe extérieure duquel est disposée respectivement au moins une saillie (14, 14a, 1a),
et dans laquelle le mélangeur comporte un boîtier définissant un espace de mélange, dans laquelle une ouverture d'évacuation est prévue à une extrémité d'évacuation du boîtier et deux embouts d'admission (3a, 3b) débouchant dans l'espace de mélange, dont les axes longitudinaux s'étendent parallèlement l'un à l'autre, sont prévus à son extrémité d'admission opposée du mélangeur,
**caractérisée en ce que** le mélangeur (3) comporte un filetage extérieur (45) et la cartouche double (1) comporte un filetage intérieur (44) pour la fixation du mélangeur (3) sur la cartouche double (1),
**en ce que** l'au moins une saillie (14, 14a, 1a) est disposée de telle façon que les saillies forment conjointement une plaque (1a) et **en ce qu'**au moins un canal de guidage (14b) est formé entre les saillies (14, 14a, 1a), lequel est défini par une ouverture dans la plaque (1a) et s'étend parallèlement aux axes longitudinaux des embouts d'évacuation (6, 7),
**en ce qu'**à l'extrémité d'admission du mélangeur (3), il est prévu au moins une nervure de guidage (13) s'étendant parallèlement aux axes longitudinaux des embouts d'admission (3a, 3b),
et **en ce que** la largeur du canal de guidage (14b) formé entre les saillies (14, 14a, 1a) et la largeur de l'au moins une nervure de guidage (13) sont adaptées l'une à l'autre pour guider le mélangeur (3) dans la cartouche double (1).

2. Combinaison selon la revendication 1, **caractérisée en ce que** les longueurs des embouts d'admission (3a, 3b), de la nervure de guidage (13) et des embouts d'évacuation (6, 7) ainsi que la position des saillies (14, 14a, 1a) sont adaptées de telle façon les unes aux autres, que lors de la pose du mélangeur (3) sur la cartouche double (1), la nervure de guidage (13) s'engage dans le canal de guidage (14b) formé entre les saillies (14, 14a), avant que les embouts d'admission (3a, 3b) touchent les embouts d'évacuation (6, 7).

3. Combinaison selon la revendication 1 ou 2, **caractérisée en ce que** la largeur de la nervure de guidage (13) est supérieure à la section transversale d'ouverture du plus grand embout d'évacuation (6) de la cartouche double (1).

4. Combinaison selon l'une des revendications 1 à 3, **caractérisée en ce qu'**une saillie de guidage (14a) facilitant l'insertion de la nervure de guidage (13) dans le canal de guidage (14b) formé comme une fente est formée sur la plaque (1a).

5. Combinaison selon la revendication 4, **caractérisée en ce que** la saillie de guidage (14a) comporte deux régions convergeant l'une vers l'autre en forme d'entonnoir, lesquelles sont prévues de part et d'autre du canal de guidage (14b) et reliées entre elles par une section en forme d'arc.

6. Combinaison selon la revendication 4 ou 5, **caractérisée en ce que** des nervures opposées au canal de guidage (14b) sont prévues sur la plaque (1a).

7. Mélangeur destiné à être utilisé dans une combinaison selon l'une des revendications précédentes, avec un boîtier définissant un espace de mélange, dans laquelle une ouverture d'évacuation est prévue à une extrémité d'évacuation du boîtier et deux embouts d'admission (3a, 3b) débouchant dans l'espace de mélange, dont les axes longitudinaux s'étendent parallèlement l'un à l'autre, sont prévus à son extrémité d'admission opposée du mélangeur, **caractérisé en ce qu'**au moins une nervure de guidage (13) s'étendant parallèlement aux axes longitudinaux des embouts d'admission (3a, 3b) est prévue à l'extrémité d'admission, et e, ce qu'au moins un élément de verrouillage formé comme un filetage extérieur (45) est prévu à l'extrémité d'admission pour la fixation détachable sur un système de cartouches.

8. Mélangeur selon la revendication 7, **caractérisé en ce que** le filetage extérieur (45) est formé par plusieurs âmes de segment de filetage non superposées.

9. Mélangeur selon la revendication 7 ou 8, **caractérisé en ce que** dans une direction opposée à l'extrémité d'évacuation, la longueur des embouts d'admission (3a, 3b) est inférieure à la longueur de l'au moins une nervure de guidage (13).

10. Mélangeur selon l'une des revendications 7 à 9, **caractérisé en ce que** les embouts d'admission (3a, 3b) et l'au moins une nervure de guidage (13) font partie intégrante d'une section d'accouplement (3d) du mélangeur (3), laquelle est reçue de façon à pouvoir tourner librement dans un boîtier de mélangeur (3c).

11. Mélangeur selon l'une des revendications 7 à 10, avec un arbre de mélangeur entraîné (40) présentant un contour à six pans creux (42) pour la réception solidaire en rotation d'un arbre d'entraînement, **caractérisé en ce que** l'arbre de mélangeur (40) est pourvu d'une phase annulaire (41) du côté de l'extrémité d'admission et/ou de triangles (43) prolongeant le contour à six pans creux (42).

12. Mélangeur selon l'une des revendications 7 à 11, **caractérisé en ce que** les embouts d'admission (3a, 3b) débouchent sur des plans décalés les uns par rapport aux autres dans l'espace de mélange formé dans le boîtier de mélangeur (3c).

13. Mélangeur selon la revendication 12 avec un arbre de mélangeur entraîné (40), **caractérisé en ce que** les deux plans dans lesquels entrent les masses sont séparés l'un de l'autre par un disque (50) sur l'arbre de mélangeur (40), des ouvertures (51) permettant le passage de la masse entrant par l'un des embouts d'admission (3b).

14. Mélangeur selon la revendication 13, **caractérisé en ce que** des nervures (52) sont prévues dans les espaces intermédiaires entre les ouvertures (51).

15. Mélangeur selon l'une des revendications 7 à 14 avec un arbre de mélangeur entraîné (40), **caractérisé en ce qu'**un espace annulaire entourant l'arbre de mélangeur (40) est formé à l'intérieur du mélangeur (3), dans lequel la masse entrant par l'un des embouts d'admission (3b) peut être recueillie avant d'arriver dans l'espace de mélange.
